(19) Europäisches Patentamt
European Patent Office
Office européen des brevets

(11) Veröffentlichungsnummer: **0 180 813**
**B1**

(12) # EUROPÄISCHE PATENTSCHRIFT

(45) Veröffentlichungstag der Patentschrift: **01.06.88**

(21) Anmeldenummer: **85112989.0**

(22) Anmeldetag: **14.10.85**

(51) Int. Cl.[4]: **C 07 D 239/82,** C 07 D 403/10, C 07 D 401/06, C 07 D 401/12, C 07 D 413/06, C 07 D 403/06, B 41 M 5/12, C 07 C 125/065, C 07 C 127/22, C 09 B 11/26, C 09 B 11/24 // (C07D403/10, 239:00, 231:00), (C07D401/06, 239:00, 213:00), (C07D401/06, 239:00, 215:00), (C07D401/12, 239:00, 211:00), (C07D413/06, 263:00, 239:00), (C07D403/06, 239:00, 209:00)

(54) **Chromogene 4,4-Diaryl-dihydrochinazolone, ihre Herstellung und Verwendung.**

(30) Priorität: **26.10.84 DE 3439282**

(43) Veröffentlichungstag der Anmeldung: **14.05.86 Patentblatt 86/20**

(45) Bekanntmachung des Hinweises auf die Patenterteilung: **01.06.88 Patentblatt 88/22**

(84) Benannte Vertragsstaaten: **CH DE FR GB LI**

(56) Entgegenhaltungen: **US - A - 4 074 050**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Berneth, Horst, Dr., Erfurter Strasse 1, D-5090 Leverkusen (DE)**
Erfinder: **Brack, Alfred, Dr., Auf dem Krahwinkel 7, D-5068 Odenthal (DE)**
Erfinder: **Meisel, Karlheinrich, Dr., Carl-von-Ossietzky-Strasse 24, D-5090 Leverkusen (DE)**

EP 0 180 813 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der Erfindung sind chromogene 4,4-Diaryldihydrochinazolone der Formel I

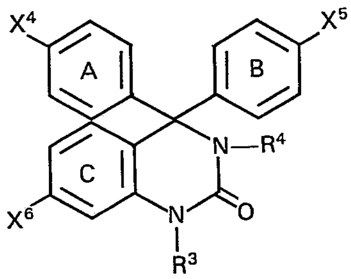

(I)

worin

einer der Reste $X^4$, $X^5$ oder $X^6$ für $NY^4Y^5$ und die anderen unabhängig voneinander Wasserstoff, Halogen, $C_1$- bis $C_{18}$-Alkyl, gegebenenfalls durch Chlor und/oder $C_1$- bis $C_{12}$-Alkyl substituiertes Phenyl, $C_1$- bis $C_{12}$-Alkanoylamino, gegebenanfalls durch Chlor und/oder $C_1$- bis $C_{12}$-Alkyl substituiertes Benzoylamino, gegebenenfalls durch $C_1$- bis $C_4$-Alkyl, $C_1$- bis $C_4$-Alkoxy, Chlor oder Phenyl substituierte Indolyl- oder Piperidylreste, $NY^4Y^5$, $OY^6$ oder $SY^6$,

$R^3$ Wasserstoff, $C_1$- bis $C_{18}$-Alkyl, Cyclohexyl oder gegebenenfalls durch Chlor und/oder $C_1$- bis $C_{18}$-Alkyl substituierte Benzyl- oder Phenylreste,

$R^4$ einen Rest der Formeln

-CN, 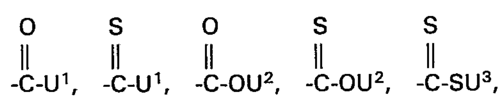

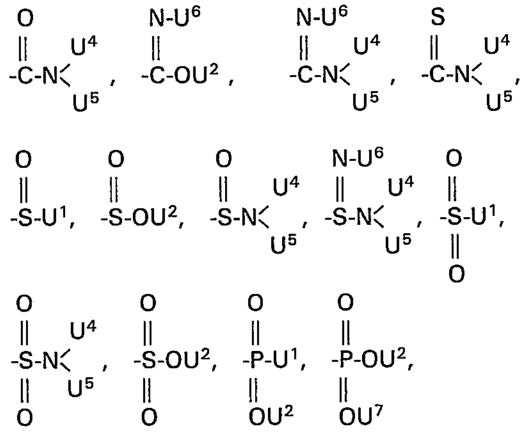

$U^1$ bis $U^7$ Wasserstoff, gegebenenfalls Fluor, Chlor, Cyano, $C_1$- bis $C_4$-Alkoxycarbonyl und/oder $C_1$- bis $C_4$-Alkoxy tragendes $C_1$- bis $C_{30}$-Alkyl, gegebenenfalls Chlor und/oder $C_1$- bis $C_{18}$-Alkyl tragendes Cyclohexyl, gegebenenfalls Chlor, Brom, Nitro, $C_1$- bis $C_{18}$-Alkyl, $C_1$-$C_{18}$-Alkoxy, $C_1$- bis $C_{18}$-Alkylthio, $C_1$-$C_{18}$-Mono- oder -Dialkylamino, $C_1$- bis $C_{18}$-Alkylsulfonyl, Cyano und/oder $C_1$- bis $C_{18}$-Alkoxycarbonyl tragende Phenyl-, Benzyl-, Naphthyl-, Picolyl-, Pyridyl-, Chinolyl-, Pyrimidyl-, Pyrazinyl-, Triazolyl-, Thiadiazolyl-, Tetrazolyl-, Indolyl-, gegebenenfalls benzanellierte Imidazol-, Oxazol- oder Thiazolreste,

$Y^4$, $Y^5$ und $Y^6$ unabhängig voneinander Wasserstoff, gegebenenfalls durch Chlor, Cyano, $C_1$- bis $C_4$-Alkoxycarbonyl oder $C_1$- bis $C_4$-Alkoxy substituiertes $C_1$- bis $C_{18}$-Alkyl, Cyclohexyl oder Phenyl oder Benzyl, die durch Chlor, $C_1$- bis $C_{12}$-Alkyl oder $C_1$- bis $C_{12}$- Alkoxy substituiert sein können, durch $C_1$- bis $C_4$-Alkyl substituiertes Piperidyl oder Glieder, die zusammen mit N oder O, an die sie gebunden sind und einem der Ringe A, B oder C zur Vervollständigung eines Ringsystems der folgenden Formeln

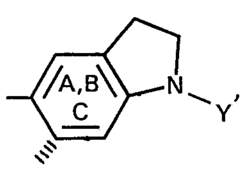, 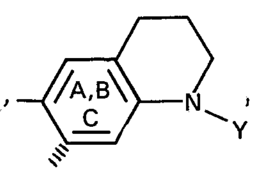, 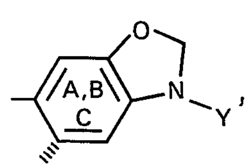, 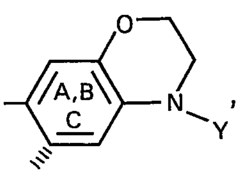,

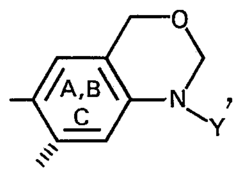, 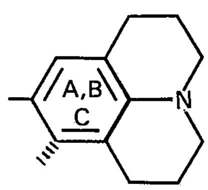, 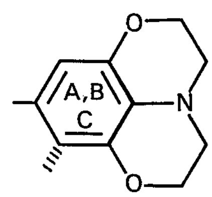, 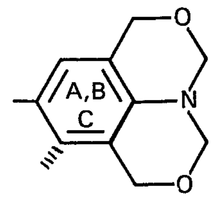,

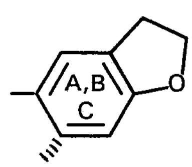, 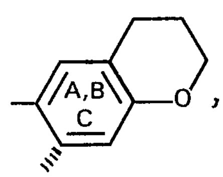, 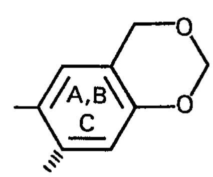, 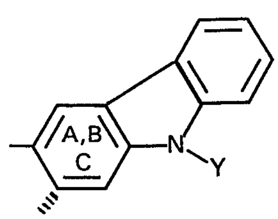

erforderlich sind, bedeuten, worin die gestrichelte Linie die weitere Anellierung im Fall von Ring C bedeutet,

Y für Wasserstoff, $C_1$- bis $C_{18}$-Alkyl, das durch Chlor, Cyano, $C_1$- bis $C_4$-Alkoxycarbonyl oder $C_1$- bis $C_4$-Alkoxy substituiert sein kann, Cyclohexyl, Phenyl oder Benzyl, die durch Chlor, $C_1$- bis $C_{12}$-Alkyl oder $C_1$- bis $C_{12}$-Alkoxy substituiert sein können, steht,

der gesättigte Ringteil bis zu 4 Reste aus der Gruppe Chlor, $C_1$- bis $C_4$-Alkyl, $C_1$- bis $C_4$-Alkoxy oder Phenyl tragen kann,

die Ringe A, B und C durch Chlor, $C_1$- bis $C_4$-Alkyl, $C_1$- bis $C_4$-Alkoxy, gegebenenfalls durch Chlor, $C_1$- bis $C_4$-Alkyl oder $C_1$- bis $C_4$-Alkoxy substituiertes Phenoxy oder Phenylamino und/oder $C_1$- bis $C_4$-Alkanoylamino substituiert sein können, oder

$NY^4Y^5$ einen gegebenenfalls durch Chlor, $C_1$- bis $C_4$-Alkyl oder Phenyl substituierten Pyrrolo-, Pyrrolidino-, Piperidino-, Pipecolino-, Morpholino-, Pyrazolo- oder Pyrazolinorest bedeutet,

ihre Herstellung und ihre Verwendung in druckkopierfähigen, thermoreaktiven und elektrochromen Aufzeichnungsmaterialen.

Unter Halogen ist im besonderen Chlor und Brom zu verstehen.

Die Phenyl- und Naphthylreste und die Reste in Benzyl- oder Benzoylaminogruppen können bis zu 3 gleiche oder verschiedene Reste tragen.

Besondere Beispiele für substituierte Phenylreste sind 2-, 3- oder 4-Tolyl, 2-, 3- oder 4-Anisyl, 2-, 3- oder 4-Chlor-phenyl, 2-, 3- oder 4-Nitro-phenyl, 2- 3- oder 4-Cyano-phenyl, 2-, 3- oder 4-Ethoxycarbonylphenyl, 2-, 3- oder 4-Methoxysulfonyl-phenyl, 2-, 3- oder 4-Trifluormethyl-phenyl, 2,3-Dinitrophenyl, 3,4-Dimethylphenyl, 2-Chlor-4-nitro-phenyl, 3-Chlor-4-nitro-phenyl, 5-Chlor-2-methyl-4-nitro-phenyl, 4-Chlor-3-methylphenyl, 3-Chlor-4-methoxy-phenyl, 3-Chlor-4-trifluormethyl-phenyl, 3,4-Dicyano-phenyl, 2,5-Dichlor-4-cyano-phenyl, 2-Methyl-1-naphthyl.

Die heterocyclischen Reste können bis zu 4 gleiche oder verschiedene Reste tragen. Besondere Beispiele für substituierte heterocyclische Reste sind 2-Methyl-4-pyridyl, 4-Nitro-2-pyridyl, 4-Phenyl-thiazol-2-yl, 5-Methyl-benzoxazolyl, 5-tert.-Butyl-benzthiazolyl, 1,2-Dimethyl-indol-3- oder -5-yl, 2,2,6,6-Tetramethyl-piperidin-4-yl.

Beispiele für im gesättigten Ring substituierte Reste sind:

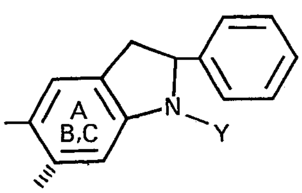
,
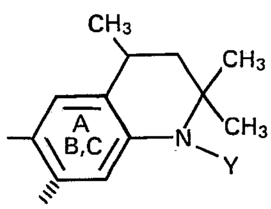
,
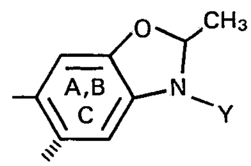
,

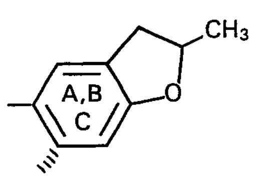
,
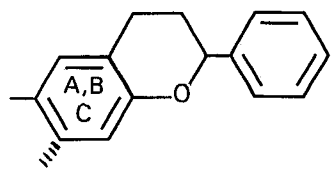
und
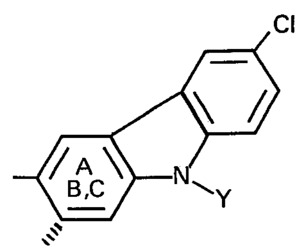

Bevorzugt sind Verbindungen der Formel II

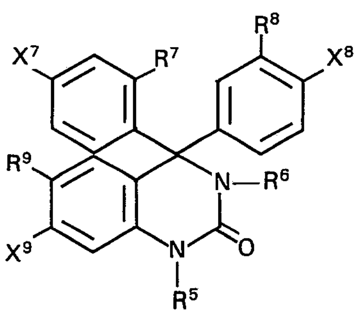
(II)

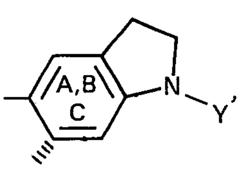
,
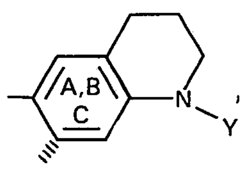
,
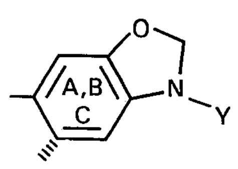
,
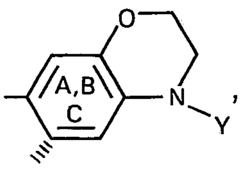
,

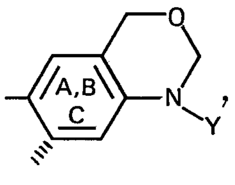, 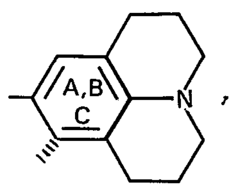, 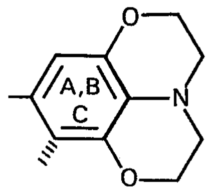, 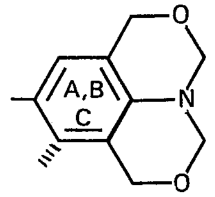,

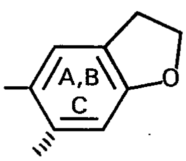, 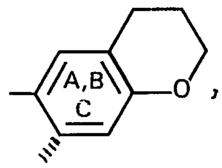, 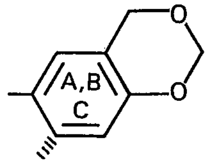, 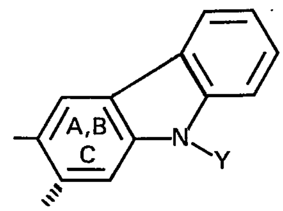

worin
die gestrichelte Linie die weitere Anellierung im Fall von Ring C bedeutet,

Y für Wasserstoff, $C_1$- bis $C_{18}$-Alkyl, das durch Chlor, Cyano, $C_1$- bis $C_4$-Alkoxycarbonyl oder $C_1$- bis $C_4$-Alkoxy substituiert sein kann, Cyclohexyl, Phenyl oder Benzyl, die durch Chlor, $C_1$- bis $C_{12}$ -Alkyl oder $C_1$- bis $C_{12}$-Alkoxy substituiert sein können, steht,

der gesättigte Ringteil bis zu 4 Reste aus der Gruppe Chlor, $C_1$- bis $C_4$-Alkyl, $C_1$ bis $C_4$-Alkoxy oder Phenyl tragen kann,

die Ringe A, B und C durch Chlor, $C_1$- bis $C_4$-Alkyl, $C_1$- bis $C_4$-Alkoxy, gegebenenfalls durch Chlor, $C_1$- bis $C_4$-Alkyl oder $C_1$- bis $C_4$-Alkoxy substituiertes Phenoxy oder Phenylamino und/oder $C_1$- bis $C_4$-Alkanoylamino substuiert sein können, oder

$NY^4Y^5$ einen gegebenenfalls durch Chlor, $C_1$- bis $C_4$-Alkyl oder Aryl, insbesondere Phenyl, substituierten Pyrrolo-, Pyrrolidino-, Piperidino-, Pipecolino-, Morpholino-, Pyrazolo- oder Pyrazolinorest bedeuten.

Beispiele für im gesättigten Ring substituierte Reste sind:

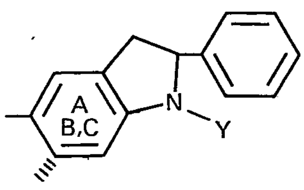, 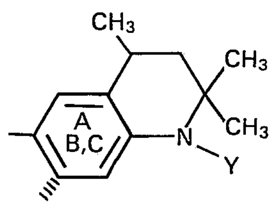, 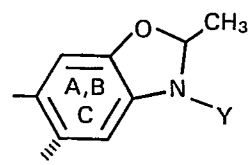,

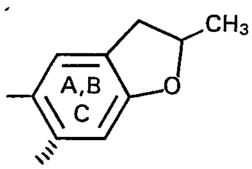, 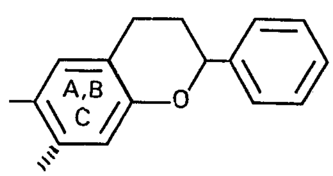 und 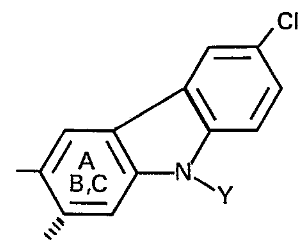

In bevorzugten Verbindungen der Formel

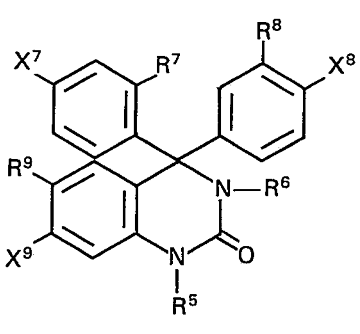

(IV)

worin
einer der Reste $X^7$, $X^8$ und $X^9$ für $NY^7Y^8$ steht und die anderen unabhängig voneinander Wasserstoff, Chlor, Brom, $C_1$- bis $C_{18}$-Alkyl, gegebenenfalls durch Chlor und/oder $C_1$- bis $C_4$-Alkyl substituiertes Phenyl, $C_1$- bis $C_4$-Alkanoylamino, gegebenenfalls durch Chlor und/oder $C_1$- bis $C_4$-Alkyl substituiertes Benzoylamino, gegebenenfalls durch methyl, Ethyl, Methoxy, Chlor oder Phenyl substituierte Indolyl- oder Piperidylreste, $NY^7$ $Y^8$, $OY^9$ oder $SY^9$,

$R^5$ Wasserstoff, $C_1$- bis $C_4$-Alkyl, Cyclohexyl oder Phenyl,

$R^6$ 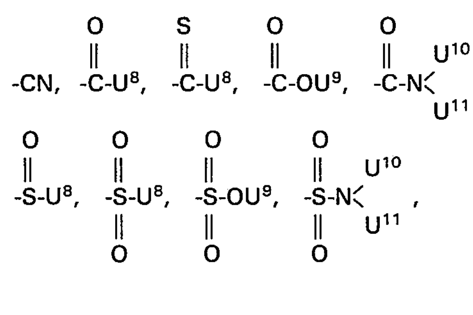

$U^8$-$U^{11}$ Wasserstoff, gegebenenfalls Fluor, Chlor oder $C_1$- bis $C_4$-Alkoxy tragendes $C_1$- bis $C_{18}$-Alkyl, Cyclohexyl, gegebenenfalls Chlor und/oder $C_1$- bis $C_8$-Alkyl tragendes Benzyl, gegebenenfalls Chlor, Brom, Nitro, $C_1$- bis $C_{18}$-Alkyl, $C_1$- bis $C_{18}$-Alkoxy, $C_1$- bis $C_{18}$-Alkylthio, $C_1$- bis $C_{18}$-Dialkylamino, $C_1$- bis $C_{18}$-Alkylsulfonyl, Cyano und/oder $C_1$- bis $C_{18}$-Alkoxycarbonyl tragende Phenyl-, Naphthyl-, Picolyl-, Pyridyl-, Chinolyl-, Pyrimidyl-, Pyrazinyl-, Triazinyl-, Triazolyl-, Thiadiazolyl-, Tetrazolyl-, Indolyl-, gegebenenfalls benzanellierte Imidazol-, Oxazol- oder Thiazolreste,

$R^7$, $R^8$ und $R^9$ unabhängig voneinander Wasserstoff, Chlor, Brom, $C_1$- bis $C_4$-Alkyl, $C_1$- bis $C_4$-Alkoxy, $C_1$- bis $C_4$-Alkylthio, Amino oder $C_1$- bis $C_4$-Alkanoylamino, $C_1$- bis $C_4$-Mono- oder -Dialkylamino, gegebenenfalls durch Methyl, Methoxy, Ethoxy oder Chlor substituiertes Anilino oder N-$C_1$- bis $C_4$-Alkylanilino,

$Y^7$, $Y^8$ und $Y^9$ unabhängig voneinander Wasserstoff, durch Chlor, Cyano, Methoxycarbonyl, Hydroxy, Methoxy oder Ethoxy substituiertes $C_1$- bis $C_{18}$-Alkyl; Cyclohexyl, Benzyl oder Phenyl, die durch Chlor, Methyl oder Methoxy substituiert sein können, 2,2,6,6-Tetramethyl- oder 1,2,2,6,6--Pentamethyl-piperidin-4-yl bedeuten oder

$Y^7$, $Y^8$ und $Y^9$ für die Glieder stehen, die zu den bei Formel I unter $Y^4$, $Y^5$ bzw. $Y^6$ aufgeführten Ringsystemen führen, oder

$NY^7Y^8$ für einen durch $C_1$- bis $C_4$-Alkyl und/oder Phenyl substituierten Pyrrolidino-, Piperidino-, Morpholino- oder Pyrazolinorest stehen.

Ganz besonders zu nennen sind 4,4-Diaryldihydrochinazolone der Formel III

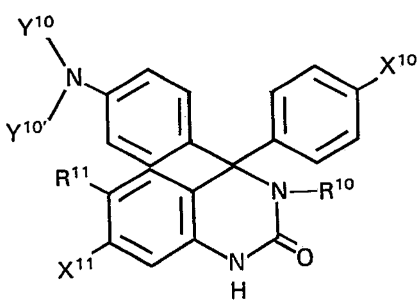

(III)

worin

$R^{10}$ Cyano, Acetyl, Trifluoracetyl, Propionyl, Butanoyl, Pivaloyl, Hexanoyl, Octanoyl, 2-Ethylhexanoyl, Decanoyl, Dodecanoyl, Tetradecanoyl, Hexadecanoyl, Octadecanoyl, Cyclohexylcarbonyl, Phenylacetyl, Benzoyl, Chlorbenzoyl, Toluoyl, tert.-Butyl-benzoyl, Hexylbenzoyl, Dodecylbenzoyl, Anisoyl, Nitrobenzoyl, Cyanobenzoyl, Trifluormethylbenzoyl, Methoxycarbonylbenzoyl, Hexoxycarbonylbenzoyl, Dodecoxycarbonylbenzoyl, Methoxysulfonylbenzoyl, Methylsulfonylbenzoyl, Dinitrobenzoyl, Chlor-nitro-benzoyl, Dichlor-benzoyl, Chlorethylthiobenzoyl, Naphthoyl, Pyridoyl, Chinoloyl, 2-, 3- oder 5-Indoloyl, 5-Benzoxazoloyl, Pyridylacetyl, Methoxycarbonyl, Ethoxycarbonyl, Propoxycarbonyl, Butoxycarbonyl, Hexoxycarbonyl, Dodecoxycarbonyl, Octadecoxycarbonyl, Phenoxycarbonyl, Dimethylaminocarbonyl, Diethylaminocarbonyl, Phenylaminocarbonyl, Methylphenylaminocarbonyl, Dimethylaminosulfonyl, Diethylaminosulfonyl, Phenylaminosulfonyl, Methylphenylaminosulfonyl, Methansulfonyl, Ethansulfonyl, Trifluormethansulfonyl, Nonafluorbutansulfonyl, Heptadecafluoroctansulfonyl, Methansulfinyl, Octansulfinyl, Benzolsulfonyl, Chlorbenzolsulfonyl, Dichlorbenzolsulfonyl, Nitro-benzolsulfonyl, Chlor-nitro-benzolsulfonyl, Trichlor-nitro-benzolsulfonyl, Toluolsulfonyl, Chlor-methyl-benzolsulfonyl, Methylnitro-benzolsulfonyl, Dimethyl-benzolsulfonyl, Methyl-dinitro-benzolsulfonyl, Methoxybenzolsulfonyl, Dichlor-methoxy-benzolsulfonyl, Methoxynitrobenzolsulfonyl, Naphthalinsulfonyl, Biphenylsulfonyl, Cyano-biphenylsulfonyl, Benzolsulfinyl, Chlorbenzolsulfinyl, Methyl-benzolsulfinyl, Benzoxazol-5-sulfonyl

$R^{11}$ Wasserstoff, Chlor, Methyl oder Methoxy,

$X^{10}$ $N^{11}Y^{11'}$, Wasserstoff, Chlor, $C_1$- bis $C_4$-Alkyl, Methoxy, Ethoxy, Propoxy, Butoxy, Hexoxy, Dodecoxy, Octadecoxy, Benzyloxy, Methylthio oder Ethylthio,

$X^{11}$ Wasserstoff, Chlor, $C_1$- bis $C_4$-Alkyl, Benzyl, Methoxy, Ethoxy, Propoxy, Butoxy, Hexoxy, Dodecoxy, Octadecoxy, Benzyloxy, Methylthio, Ethylthio, Acetylamino, Propionylamino, Butanoylamino, Decanoylamino, Octadecanoylamino, Benzoylamino, $NHCONHR^{10}$ oder $NY^{12}Y^{12'}$ bedeuten,

$Y^{10}$-$Y^{12}$ Methyl, Ethyl, Propyl, Butyl, Hexyl, Dodecyl, Octadecyl, Cyanethyl, Methoxyethyl, Methoxycarbonylethyl, Benzyl, Phenyl, 4-Tolyl, 4-Chlorphenyl, 4-Anisyl, 4-Ethoxy-phenyl, 4-Cyano-phenyl,

$Y^{10'}$-$Y^{12'}$ Wasserstoff oder die Bedeutung von $Y^{10}$-$Y^{12}$, oder

$NY^{10}Y^{10'}$, $NY^{11}Y^{11'}$ oder $NY^{12}Y^{12'}$

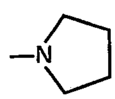 , 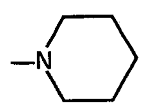 , 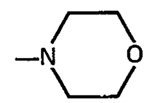 ,

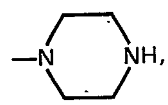 , 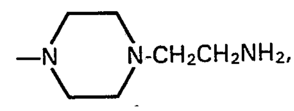 ,

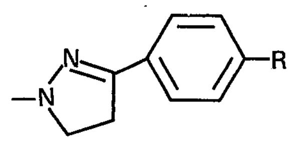 ,

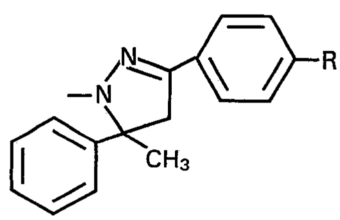 ,

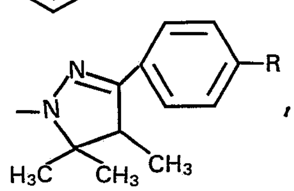 , 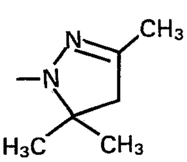

und

R Wasserstoff, Chlor, Methyl, Methoxy, Ethoxy oder Cyano bedeuten.

Ein weiterer Gegenstand der Erfindung sind Leukoverbindungen der Formel IV

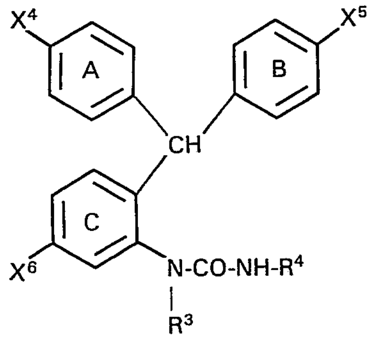

(IV)

worin

$R^3$, $R^4$, $X^4$, $X^5$, $X^6$, A, B und C die bei Formel I und bevorzugt bei den Formeln II und III angegebene Bedeutung haben.

Ein weiterer Gegenstand der Erfindung ist ein Verfahren zur Herstellung eines 4,4-Diaryl-dihydrochinazolons der Formel I, dadurch gekennzeichnet, dass man eine Harnstoffverbindung der Formel V

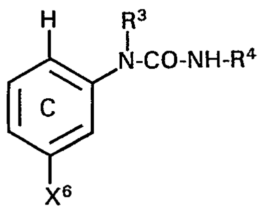

(V)

mit einem Keton der Formel VI

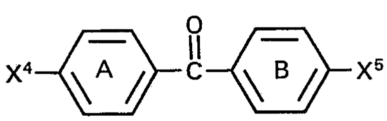

(VI)

umsetzt, worin

$X^4$, $X^5$, $X^6$, $R^3$, $R^4$, A, B und C die oben angegebene Bedeutung besitzen.

Insbesondere handelt es sich bei den Verbindungen V um solche, bei denen

$X^6$ ein Elektronendonorsubstituent ist, wie $NY^4Y^5$, $OY^6$ oder $SY^6$, worin

$Y^4$ bis $Y^6$ die oben angegebene Bedeutung besitzen und der Ring C nicht durch starke Elektronenakzeptorsubstituenten, wie z. B. Nitro, Cyano oder Alkoxycarbonyl desaktiviert ist.

Die Umsetzung erfolgt üblicherweise mit wasserabspaltenden Reagenzien ohne oder auch mit unter diesen Bedingungen inerten Lösungsmitteln bei Temperaturen zwischen 0°C und dem Siedepunkt des jeweiligen Mediums. Anschliessend wird evtl. nach Entfernen der inerten Lösungsmittel auf beispielsweise Wasser oder einen Alkohol ausgetragen. Durch Anheben des pH-Wertes mit beispielsweise Alkali- oder Erdalkalimetallhydroxiden, Carbonaten, Hydrogencarbonaten, Ammoniak oder Aminen bis zum Verschwinden der Farbe dieser Mischung erhält man die 4,4-Diaryl-dihydrochinazolone der Formel I. Hierbei kann es notwendig sein, zur Wasserabspaltung aus evtl. gebildeten Carbinolbasen einige Zeit zu erwärmen oder das primär erhaltene unsaubere Produkt in Lösungsmitteln, wie Alkoholen — beispielsweise Methanol, Ethanol, 2-Propanol, Butanol; Nitrilen — beispielsweise Acetonitril; Ketonen — beispielsweise Aceton, 2-Butanon oder Estern — beispielsweise Essigsäureethylester, Essigsäurebutylester, einige Zeit bei Temperaturen zwischen Raumtemperatur und der Siedetemperatur des jeweiligen Mediums zu behandeln.

Wasserabspaltende Reagenzien sind beispielsweise Phosphoroxychlorid, Phosphorpentachlorid, Diphosphorpentoxid, Phosgen, Phosphortrichlorid, Phosphortribromid, Sulfurylchlorid, Thionylchlorid, Oxalylchlorid oder Mischungen hiervon. Vorzugsweise werden Phosphoroxychlorid und Phosphoroxychlorid/Diphosphorpentoxid eingesetzt.

Geeignete inerte Lösungmittel sind beispielsweise Toluol, Chlorbenzol, Dichlorbenzol, Nitrobenzol, chlorierte aliphatische Kohlenwasserstoffe, wie 1,2-Dichlor-ethan.

Die Herstellung der 4,4-Diaryl-dihydrochinazolone der Formel I kann auch durch Oxidation von Leukoverbindungen der Formel IV erfolgen.

Diese Oxidation kann auf bekannte Weise mit höherwertigen Metallverbindungen, wie $PbO_2$, $MnO_2$, Permanganaten, $CrO_3$, Chromaten, Dichromaten, $NiO_2$, $K_3$ $[Fe(CN)_6]$, mit Chinonen, wie Chloranil, Tetrachlor-o-chinon, Dichlor-dicyano-chinon, oder auf eine andere literaturbekannte Weise, wie z.B. mit Sauerstoff, Luft, Perboraten oder Wasserstoffperoxid, erfolgen.

Die Aufarbeitung, Isolierung und evtl. Nachbehandlung erfolgt auf analoge Weise wie oben beschrieben.

Die Oxidation mit höherwertigen Metallverbindungen erfolgt üblicherweise in saurem Medium oder in organischen Lösungsmitteln, wie Alkoholen — z.B. Ethanol, Isopropanol, Ethylenglykolmonomethylether; Ketonen — z.B. Aceton, Butanon oder Metallisopropylketon oder polaren aprotischen Lösungsmitteln, z.B. N-Methyl-pyrrolidon, γ-Butyrolacton, Acetonitril, Dimethylsulfoxid oder Sulfolan oder in Mischungen solcher Lösungsmittel mit Säuren bei Temperaturen zwischen 0°C und 60°C, vorzugsweise 10-40°C.

Geeignete Säuren sind beispielsweise Salzsäure, Schwefelsäure, Phosphorsäure, Essigsäure, Propionsäure oder Mischungen untereinander und/oder Mischungen mit Wasser. Eine bevorzugte Mischung ist Salzsäure, Essigsäure und Wasser.

Die Oxidation mit Chinonen erfolgt üblicherweise in organischen Lösungsmitteln, wie Alkoholen — z.B. Methanol, Ethanol, Isopropanol; Ketonen — z.B. Aceton, Butanon; Estern z.B. Essigsäureethyl- oder -butylester; Carbonsäuren — z.B. Essigsäure, Propionsäure oder polaren aprotischen Lösungsmitteln, wie N-Methylpyrrolidon, Dimethylformamid, γ-Butyrolacton, Acetonitril, Sulfolan oder aber in Mischungen hiervon bei Temperaturen zwischen 0°C und dem Siedepunkt des Mediums, vorzugsweise 20-70°C.

Die 4,4-Diaryl-dihydrochinazolone der Formel II sind normalerweise farblos oder höchstens schwach gefärbt.

Wenn diese Farbbildner mit einem vorzugsweise sauren Entwickler, d.h. einem Elektronenakzeptor in Kontakt gebracht werden, so ergeben sich intensive blaue, grünblaue, grüne, violette oder rote Farbtöne, die ausgezeichnet sublimations- und lichtecht sind. Durch Mischungen untereinander lassen sich marineblaue, graue oder schwarze Färbungen erzielen.

Sie sind auch wertvoll im Gemisch mit einem oder mehreren anderen bekannten Farbbildern, z.B. 3,3-Bis-(aminophenyl)-phthaliden, 3,3-Bis-(indolyl)-phathaliden, 3-Amino-fluoranen, 2,6-Diamino-fluoranen, Leukoauraminen, Spiropyranen, Spirodipyranen, Chromenoindolen, Phenoxazinen, Phenothiazinen, Carbazolyl-methanen oder anderen Triarylmethanleukofarbstoffen, um grüne, violette, blaue, marineblaue, graue oder schwarze Färbungen zu ergeben.

Die 4,4-Diaryldihydrochinazolone der Formel I zeigen sowohl auf phenolischen Unterlagen wie auch besonders auf aktivierten Tonen eine gute Farbintensität und Lichtechtheit. Sie eignen sich vor allem als Farbbildner für die Verwendung in einem wärmeempfindlichen oder druckempfindlichen Aufzeichnungsmaterial, das sowohl Kopier- als auch Registriermaterial sein kann. Ihre Entwicklungsgeschwindigkeit ist in Abhängigkeit von den Substituenten unterschiedlich. Im allgemeinen zeichnen sie sich jedoch durch eine hohe Entwicklungsgeschwindigkeit aus bei gleichzeitig reduzierter Empfindlichkeit der Aufzeichnungsmaterialien gegenüber unbeabsichtigter vorzeitiger Entwicklung.

Ein druckempfindliches Material besteht beispielsweise aus mindestens 1 Paar von Blättern, die mindestens einen Farbbildner der Formel I, gelöst oder dispergiert in einem nichtflüchtigen organischen Lösungsmittel, und einen Elektronenakzeptor als Entwickler enthalten.

Typische Beispiele für solche Entwickler sind anorganische Stoffe wie Tone, Metallsalze oder -oxide oder organische Polymerisate wie Phenolharze.

Die Entwickler können zusätzlich auch im Gemisch mit anderen an sich unreaktiven oder wenig reaktiven Pigmenten eingesetzt werden.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen. Verfahren zur Herstellung derartiger Mikrokapseln sind bekannt.

Als nichtflüchtige Lösungsmittel sind z.B. partiell hydriertes Terphenyl, alkylierte Naphthaline oder Dibutylphthalat geeignet.

Bevorzugt wird eine Anordnung, bei der der eingekapselte Farbbildner in Form einer Schicht auf der Rückseite eines Übertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Verbindungen der Formel I können vorzugsweise auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel.

Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrokardiographen, verwendet. Die Bilderzeugung (Markierung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst oder dispergiert ist. Eine andere Möglichkeit besteht darin, dass sowohl der Farbbildner als auch der Entwickler in einer Schicht dispergiert sind. Das Bindemittel wird in spezifischen Bereichen mittels Wärme erweicht und an diesen Stellen, an denen Wärme angewendet wird, kommt der Farbbildner mit dem Elektronenakzeptor in Kontakt, und es entwickelt sich sofort die erwünschte Farbe.

Als Entwickler eignen sich die gleichen Elektronenakzeptoren wie sie in druckempfindlichen Papieren verwendet werden, vorzugsweise phenolische Verbindungen, wie sie beispielsweise in der DE-PS 1 251 348 beschrieben sind, sowie Borsäure und organische, vorzugsweise aliphatische Dicarbonsäuren.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die 4,4-Diaryl-dihydrochinazolone und der Entwickler in Wasser schwerlöslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine und Stärke.

Die thermoreaktiven Schichten können weitere Zusätze enthalten: zur Verbesserung des Weissgrades, zur Erleichterung des Bedruckens der Papiere, zur Verhinderung des Festklebens der erhitzten Feder und zur Farbbildung nur innerhalb eines begrenzten Temperaturbereiches.

Die Leukoverbindungen der Formel IV können beispielsweise als langsam entwicklelnde Farbbildner in übliche druck- oder thermoreaktive Papiere eingearbeitet werden. Dort dienen sie wegen ihrer guten Lichtechtheit als Mischkomponenten für schnell entwickelnde aber wenig lichtechte Farbbildner, z.B. Kristallviolettlacton. So wird die Lichtechtheit der Aufzeichnung verbessert.

Ausserdem sind die Leukoverbindungen der Formel IV geeignet für oxidativ-entwickelnde druck- oder thermoreaktive Aufzeichnungsmaterialien. Hier wird dem Farbbildner oder dem Elektronenakzeptor ein geeignetes Oxidationsmittel zugesetzt, die weitere Verarbeitung der Schichten ist ähnlich wie bei üblichen thermo- oder druckempfindlichen Aufzeichnungsmaterialien. Durch Kontakt mit dem Oxidationsmittel wird die Leukoverbindung zum Farbstoff oxidiert und liefert so eine gefärbte Markierung.

Die beschriebenen Verfahren und Zubereitungen sind beispielsweise aus den US-Patentschriften 2 948 753, 3 096 189 und 3 193 404 und aus den deutschen Offenlegungsschriften 2 555 080 und 2 700 937 bekannt.

Aus der US-A-4 074 050 ist die Verwendung von 4,4-Diphenyl-1,3-benzoxazin-2-onen als Farbbildner bekannt. Es handelt sich um Langsamentwickler, die zur Farbstoffentwicklung auf saurem Ton mehrere Stunden benötigen. Demgegenüber entwickeln die neuen Farbbildner den Farbstoff innerhalb von wenigen Sekunden.

Die 4,4'-Diaryldihydrochinazolone der Formel I bzw. die hieraus durch Ringöffnung gebildeten Farbstoffe sind zum Anfärben von Polyacrylnitril, tannierter Baumwolle und anderen sauer modifizierten Fasern, Geweben und Pulvern geeignet.

*Beispiel 1*

27,0 g Michlers Hydrol und 31,8 g N-(3-Dimethylaminophenyl)-N'-(4-chlorbenzoyl)-harnstoff werden in 150 ml Ethanol und 3,5 ml Eisessig unter Stickstoff 30 min. refluxiert. Nach dem Erkalten wird abgesaugt, mit Ethanol gewaschen und getrocknet: 50,0 g (88% d.Th.) graubeiges Pulver vom Schmp. und der Formel

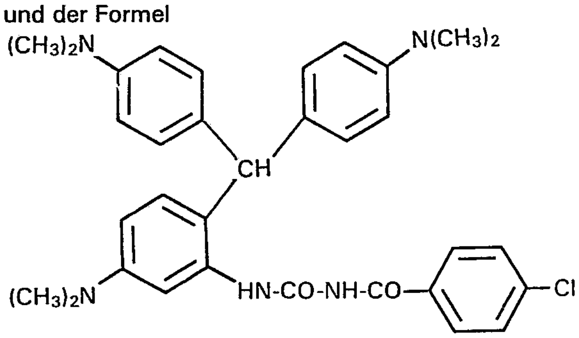

28,5 g dieser Leukoverbindung werden in 100 ml 80%iger Essigsäure und 15 g konz. Salzsäure bei 5°C mit 38 ml einer 31%igen wässrigen Bleidioxidsuspension versetzt. Die Temperatur steigt auf 32°C. Nach 7 min. werden 35 ml 20%ige Schwefelsäure zugesetzt, das Bleisulfat wird abfiltriert und das tiefblaue Filtrat nach Verdünnen mit 160 ml Methanol in eine Lösung von 80 g Natriumhydroxid in 800 ml Eiswasser eingetropft. Der blaue Niederschlag wird abgesaugt, mit Wasser neutral gewaschen. Noch feucht wird er in 100 ml Ethanol eingetragen. Die blassbeige Suspension rührt 2 h, wird abgesaugt und erneut noch feucht in 80 ml Ethanol eingetragen. Es wird 2 h bei 60-70°C gerührt, nach dem Abkühlen abgesaugt, mit Ethanol gewaschen und i.Vak. getrocknet: 20,5 g (72% der Theorie) blassbeiges Pulver vom Schmp. 261-263°C (Zers.; aus DMF) und der Formel

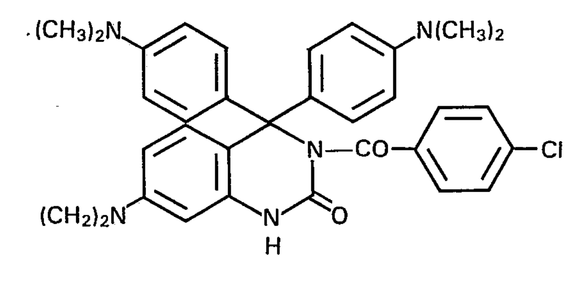

Eine Lösung in Eisessig wird blau mit $\lambda_{max}$ = 608 nm. Auf Säureton und mit Bisphenol A im Thermodruck wird eine kräftig blaue Farbe entwickelt.

Der als Ausgangsmaterial benutzte N-(3-Dimethylamino)-N'-(4-chlorbenzoyl)-harnstoff wird folgendermassen hergestellt:

Zu 27,2 g 3-Dimethylamino-anilin in 300 ml wasserfreiem Toluol tropft man 36,4 g 4-Chlorbenzoylisocyanat. Unter Erwärmung fällt ein dicker Niederschlag aus. Nach 2 h wird abgesaugt, mit Toluol gewaschen und getrocknet: 59,6 g (94% d.Th.) farbloses Kristallpulver. Dieses wird 1 h in 250 ml Ethanol gekocht, nach dem Erkalten abgesaugt, gewaschen und getrocknet: 34,4 g (54% d.Th.) farbloses Kristallpulver vom Schmp. 214,5-215,5°C und der Formel

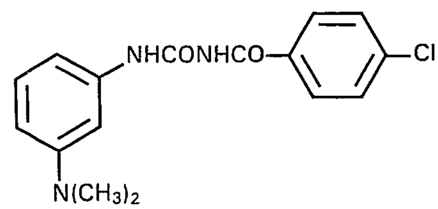

*Beispiel 2*

13,5 g Michlers Hydrol und 22,25 g N-(3-Dimethylaminophenyl)-N'-stearoyl-harnstoff werden in 70 ml Ethanol und 3 ml Eisessig und 0,5 ml konz. Salzsäure 3,5 h unter Stickstoff refluxiert. Nach dem Abkühlen wird abgesaugt, mit Ethanol gewaschen und im Vakuum getrocknet. Man erhält 26,9 g (77%) beiges Pulver vom Schmp. 123°C und der Formel

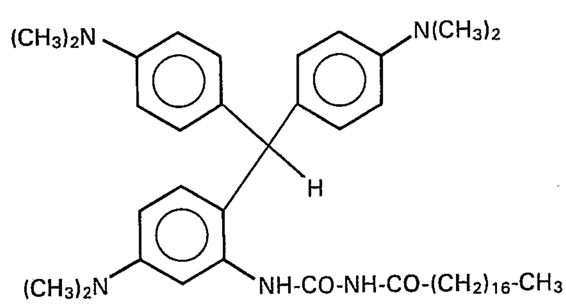

24,4 g dieser Leukoverbindung werden in 50 ml Dimethylformamid bei 50°C mit 8,61 g Chloranil versetzt. Nach 1 h bei 50-55°C wird abgekühlt, mit 200 ml Methanol verdünnt, filtriert und in eine Lösung von 24 g Natriumhydroxid in 500 ml Eiswasser eingetropft. Es wird abgesaugt, mit Wasser neutral gewaschen und bei 50°C im Vakuum getrocknet. Das Rohprodukt wird in 50 ml Ethanol unter Zusatz von 2 ml Triethylamin kurz aufgekocht, kalt gerührt, abgesaugt, mit Ethanol gewaschen und im Vakuum getrocknet: 19,4 g (80%) graues Pulver vom Schmp. 140°C und der Formel

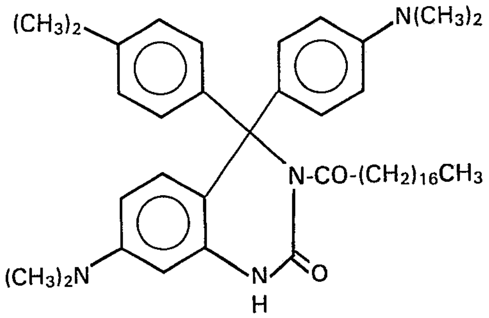

Eine Lösung in Eisessig wird blau mit $\lambda_{max}$ = 608 nm. Auf Säureton und mit Bisphenol A im Thermodruck wird eine kräftig blaue Farbe entwickelt.

Der als Ausgangsmaterial benutze N-(3-Dimethylamino)-N'-stearoyl-harnstoff wird analog zu Beispiel 1 hergestellt. Er wird in 40% Ausbeute als blassgelbes Pulver vom Schmp. 80-81°C erhalten.

Analog Beispiel 1 oder 2 werden die folgenden Dihydrochinazolone hergestellt:

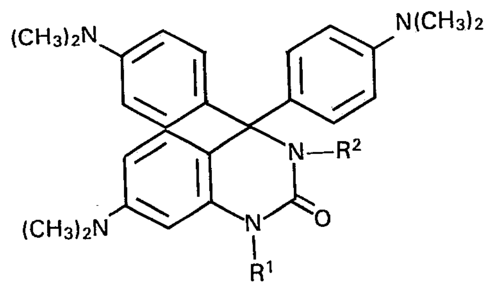

| Beispiel | $R^1$ | $R^2$ | Farbton auf Säureton oder mit Bisphenol A | $\lambda_{max}$ in nm |
|---|---|---|---|---|
| 3 | H | $-CO-C(CH_3$ | blau | 605 |
| 4 | $CH_3$ | $-CO-CH-(CH_3)_2-CH_3$<br>\|<br>$CH_2CH_3$ | türkis | 618 |
| 5 | $CH_2CH_2CN$ | 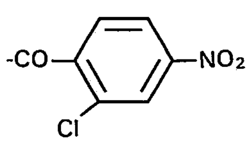 | türkis | 617 |
| 6 | H | -CN | blau | 604 |
| 7 | H | $-CO-(CH_2)_{10}CH_3$ | blau | 608 |
| 8 | 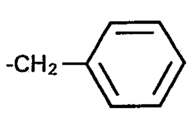 | $-CO-N(C_2H_5)_2$ | türkis | 619 |
| 9 | 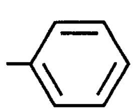 | 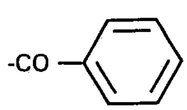 | türkis | 615 |
| 10 | H | 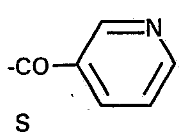 | blau | 605 |
| 11 | H | S<br>‖<br>$-C-CH_3$ | blau | 607 |
| 12 | H | $-CO-CF_3$ | blau | 604 |

| Beispiel | $R^1$ | $R^2$ | Farbton auf Säureton oder mit Bisphenol A | $\lambda_{max}$ in nm |
|---|---|---|---|---|
| 13 | H | 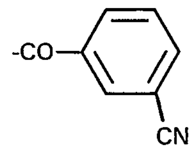 | blau | 605 |
| 14 | H | 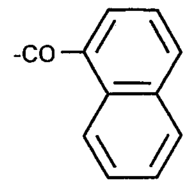 | blau | 608 |
| 15 | H | 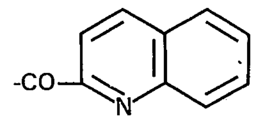 | blau | 606 |

*Beispiel 16*

270 g Michlers Hydrol und 137 g 3-Methoxy-4-methylanilin werden in 1 l Methanol und 5 ml konz. Salzsäure 2 h refluxiert. Nach dem Erkalten wird abgesaugt und aus Toluol umkristallisiert. Man erhält 230 g (59% d.Th.) beiges Kristallisat vom Schmp. 190-192°C und der Formel

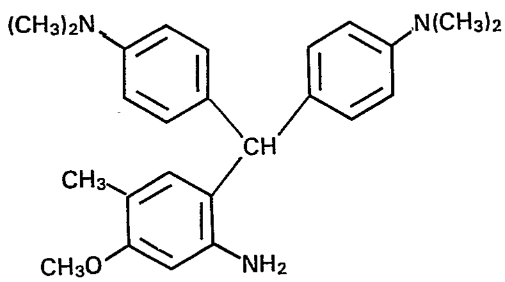

77,8 g hiervon werden in 400 ml Wasser und 57 ml konz. Salzsäure gelöst. Bei 5-10°C tropft während 1,5 h eine Lösung von 26 g Natriumcyanat in 200 ml Wasser zu. Es wird 3 h bei Raumtemperatur nachgerührt und durch Zugabe von 10%iger Salzsäure die Bildung einer Fällung verhindert. Mit Natronlauge wird auf pH = 8-9 gestellt, abgesaugt und getrocknet. Nach Umkristallisation aus Aceton erhält man 74,8 g (87% der Theorie) farbloses Kristallisat vom Schmp. 221-222°C und der Formel

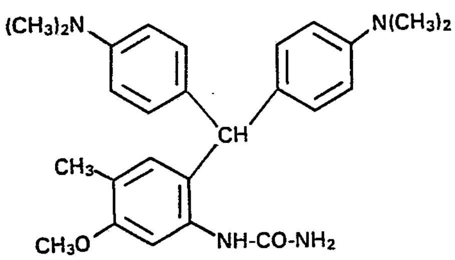

43,2 g dieses Harnstoffes werden in 100 ml Ethylenchlorid bei 60°C mit 33,3 g Stearylchlorid versetzt und anschliessend 4 h refluxiert. Das Lösungsmittel wird abgezogen und der Rückstand aus Ethanol umkristallisiert: 53,6 g (77% d.Th.) blassgrünes Kristallpulver vom Schmp. 92-93°C und der Formel

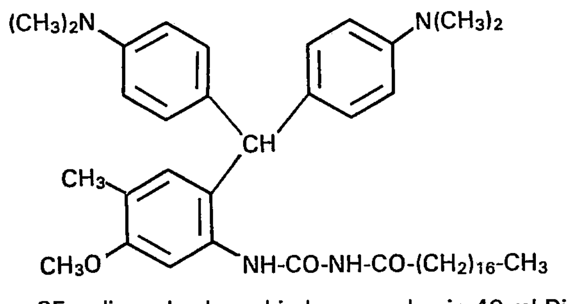

35 g dieser Leukoverbindung werden in 40 ml Dimethylformamid bei 50°C gelöst und mit 12,3 g Chloranil versetzt. Es wird 2 h bei 50°C gerührt, abgekühlt, mit 200 ml Methanol versetzt und filtriert. Das grüne Filtrat wird in eine Lösung von 8 g Natriumhydroxid in 500 ml Eiswasser eingetropft, 2 h nachgerührt, abgesaugt, neutral gewaschen und getrocknet. Dann wird in 50 ml Ethanol unter Zusatz von 1 ml Triethylamin kurz aufgekocht, abgekühlt und abgesaugt. Man erhält 12,5 g (36% d.Th.) farbloses Kristallpulver vom Schmp. 178°C und der Formel

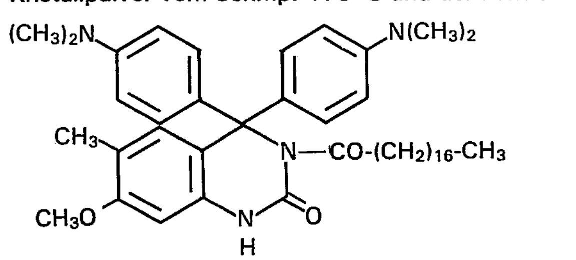

Eine Lösung in Eisessig wird blaustichig grün mit $\lambda_{max}$ = 470,622 nm. Auf Säureton und mit Bisphenol A im Thermodruck wird eine kräftige blaustichig grüne Färbung erzielt.

Unter Verwendung der entsprechenden Säurechloride werden die Beispiele 17-20 erhalten.

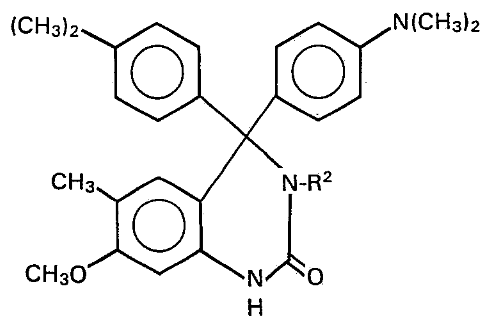

| Beispiel | $R^2$ | Schmp. | $\lambda_{max}$ nm | Farbton auf Säureton oder mit Bisphenol A |
|---|---|---|---|---|
| 17 | -CO- -Cl | 256-258°C (Zers.) | 472,622 | blaustichig grün |
| 18 | $-COC(CH_3)_3$ | 207°C | 462,622 | blaustichig grün |
| 19 | $-COCH-(CH_2)_3-CH_3$ (mit $CH_2CH_3$ an CH) | 237°C (Zers.) | 474,622 | blaustichig grün |
| 20 | $-CO(CH_2)_{10}-CH_3$ | 170-171°C | 472,622 | blaustichig grün |

*Beispiel 21*

34,3 g 3-Methoxy-4-methylanilin und 64,3 g 4-(Dimethylamino)-4'-methoxy-benzhydrol werden in 250 ml Methanol, 1,5 ml konz. Salzsäure und 30 ml Wasser 5 h refluxiert. Es wird kalt gerührt, abgesaugt, mit Methanol gewaschen und bei 40°C im Vakuum getrocknet: 53,3 g (57%) beiges Kristallisat vom Schmp. 135-137°C und der Formel

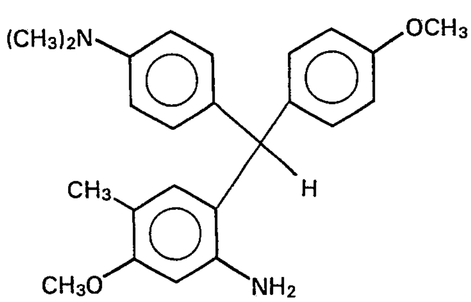

37,7 g hiervon werden in 200 ml Wasser unter Zusatz von 30 ml konz. Salzsäure bei 0-5°C gelöst. Hierzu tropft bei dieser Temperatur eine warme Lösung von 16,3 g Natriumcyanat in 200 ml Wasser. Mit Salzsäurezusatz wird der pH unter 2 gehalten. Dann wird abgesaugt, mit Eiswasser gewaschen und getrocknet. Umkristallisation aus Tetrachlorkohlenstoff ergibt 32,4 g (77%) beiges Kristallisat vom Schmp. 180-182°C und der Formel

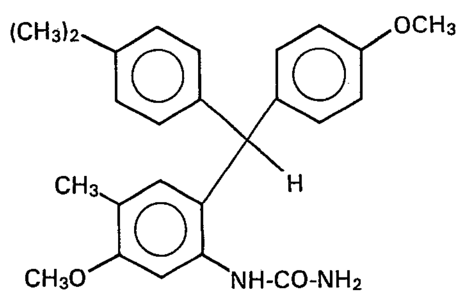

31,5 g dieses Harnstoffs werden in 75 ml Pyridin langsam mit 25,8 g Stearylchlorid bei 60°C versetzt. Nach dem Abkühlen wird in 700 ml Eiswasser ausgetragen und mit Salzsäure ein pH von 3,5 eingestellt. Über Nacht wird ausgerührt, abgesaugt, mit Wasser gewaschen und im Vakuum getrocknet. Umkristallisation aus Ethanol ergibt 34,4 g (67%) blassrosa Kristallisat vom Schmp. 79-81°C und der Formel

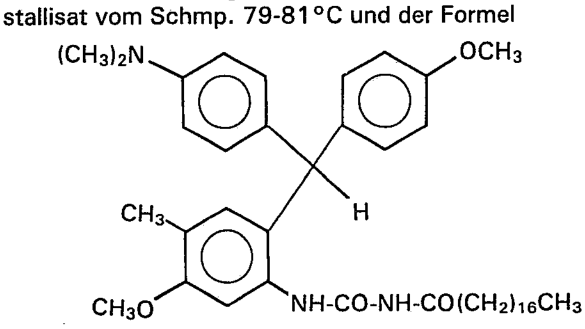

34,3 g dieser Leukoverbindung werden in 50 ml Dimethylformamid bei 60°C mit 12,3 g Chloranil versetzt. Nach 1 h bei 65-70°C wird abgekühlt, mit 200 ml Methanol verdünnt und in eine Lösung von 16 g Natriumhydroxid in 700 ml Wasser eingetropft. Die Suspension wird abgesaugt, mit 450 ml Methanol/Wasser (1:2) und 100 ml Wasser neutral gewaschen und im Vakuum getrocknet. Das Rohprodukt wird in Ethanol aufgekocht, kalt gerührt, abgesaugt, mit Ethanol gewaschen und im Vakuum getrocknet. Man erhält 17,1 g (50% d.Th.) blassrosa Kristallpulver vom Schmp. 120-122°C und der Formel

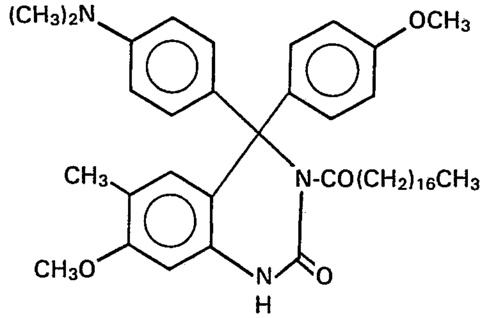

Eine Lösung in Eisessig wird tief bordo mit $\lambda_{max}$ = 380, 450, 525, 542 nm.

Auf Säureton und mit Bisphenol A wird ebenfalls eine tiefe bordo Färbung erzielt.

Analog werden die folgenden Beispiele erhalten:

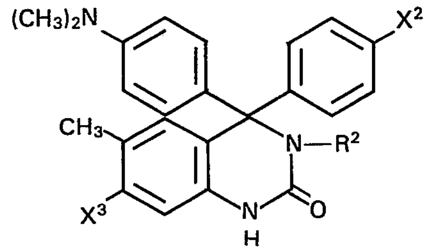

| Beispiel | $X^2$ | $X^3$ | $R^2$ | Farbton auf Säureton oder mit Bisphenol A |
|---|---|---|---|---|
| 22 | $(CH_3)_2N$ | $CH_3O$ | 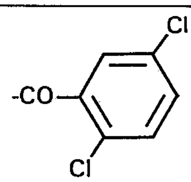 | blaustichig grün |
| 23 | $(CH_3)_2N$ | $C_{18}H_{37}O$ | 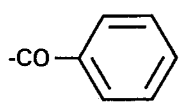 | blaustichig grün |
| 24 | $CH_3O$ | $NHC_2H_5$ | 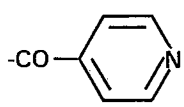 | grün |
| 25 | 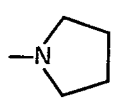 | H | $-CO-CH(CH_2)_3CH_3$<br>\|<br>$CH_2CH_3$ | gelbst. grün |
| 26 | 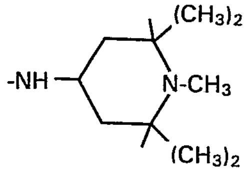 | $-C_4H_9$ | 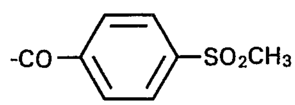 | grün |
| 27 | $(C_2H_5)_2N$ | Cl | 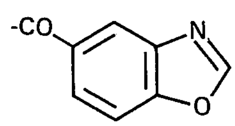 | grün |
| 28 | $(C_4H_9)_2N$ | $-NH-CO-(CH_2)_{10}CH_3$ | 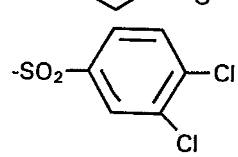 | grün |

*Beispiel 29*

68,6 g 3-Methoxy-4-methyl-anilin und 129 g 4-Dimethylamino-4'-methoxy-benzhydrol werden in 500 ml Methanol, 60 ml Wasser und 8 ml konz. Salzsäure unter Stickstoff 4 h refluxiert. Nach dem Abkühlen wird abgesaugt, mit Methanol gewaschen und getrocknet. Umkristallisation aus Methylcyclohexan ergibt 99 g (53% d.Th.) farbloses Kristallisat mit dem Schmp. 136-137°C und der Formel

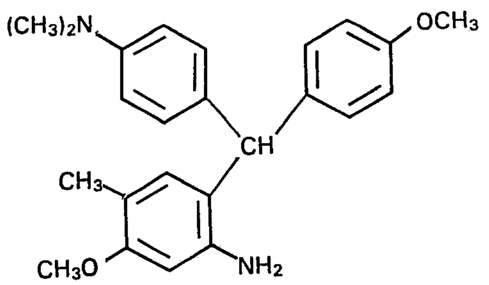

37,7 g dieser Aminoverbindung werden in 100 ml wasserfreiem Toluol bei 50°C während 30 min mit einer Lösung von 19,6 g Benzolsulfonylisocyanat in 45 ml wasserfreiem Toluol unter Stickstoff versetzt. Es wird 1 h bei 50-60°C gerührt, abgekühlt, mit 5 ml Ethanol versetzt und abgesaugt. Man erhält 36 g (92% d.Th.) rosa Kristallpulver mit dem Schmelzpunkt 198-200°C und der Formel

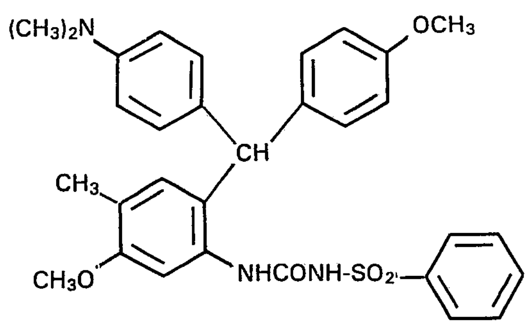

16,8 g dieser Leukoverbindung werden in 20 ml Dimethylformamid bei 60°C mit 7,4 g Chloranil versetzt. Nach 1 h bei 60°C wird abgekühlt, mit 20 ml Eisessig versetzt und auf 300 ml Eiswasser ausgetragen. Die rote Suspension wird mit 130 ml 2,5-molarer Natronlauge auf pH = 9-10 gestellt und abgesaugt. Es wird neutral gewaschen und getrocknet. Dann wird über 600 ml Kieselgel chromatographiert und mit ca. 1,5 l Chloroform/Essigester 1/1 eluiert. Nach Abziehen der Lösungsmittel wird in 35 ml Essigester aufgenommen, mit 1 ml Triethylamin versetzt und 1 h verrührt. Absaugen, Waschen mit Essigester und Trocknen ergibt 14,2 g (85% d.Th.) schwach beiges Kristallpulver vom Schmp. 174°C und der Formel

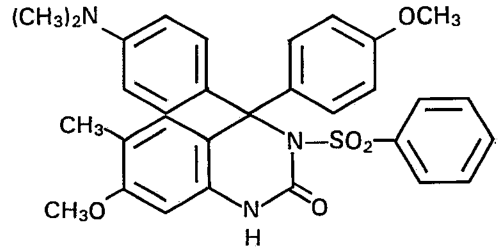

Eine Lösung in Eisessig wird tiefbordo mit $\lambda_{max}$ = 546 nm.

Auf Säureton und mit Bisphenol A im Thermodruck wird ebenfalls eine tiefrote Färbung erzielt.

Analog lassen sich die Verbindungen der Beispiele 30-41 erhalten:

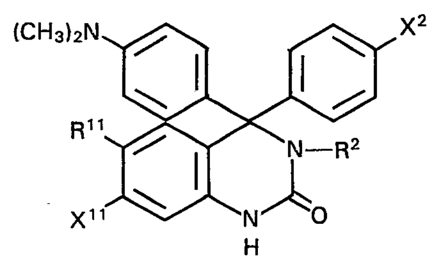

| Beispiel | $X^2$ | $X^{11}$ | $R^{11}$ | $R^2$ | Farbton auf Säureton oder mit Bisphenol A |
|---|---|---|---|---|---|
| 30 | $CH_3O$ | $CH_3O$ | $C_4H_9$ | 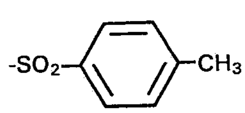 | bordo |
| 31 | $C_2H_5O$ | $CH_3O$ | $CH_3$ | 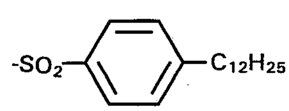 | bordo |
| 32 | Cl | $C_3H_7$ | $CH_3$ | 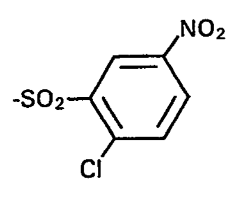 | gelb |

| Beispiel | $X^2$ | $X^{11}$ | $R^{11}$ | $R^2$ | Farbton auf Säureton mit Bisphenol A |
|---|---|---|---|---|---|
| 33 | H | $N(C_2H_5)_2$ | Cl | 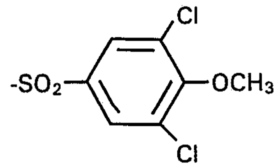 | grün |
| 34 | H | $C_6H_5CH_2O$ | $CH_3O$ | $-SO_2CF_3$ | rot |
| 35 | $CH_3$ | $N(CH_3)_2$ | $CH_3$ | 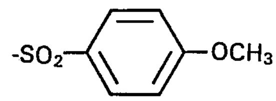 | grün |
| 36 | $CH_3O$ | H | 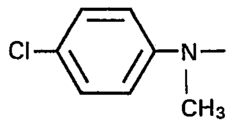 | $-SO_2CH_3$ | rot |
| 37 | $C_{18}H_{37}O$ | $C_2H_5O$ | Cl | 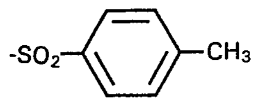 | blaustichig rot |
| 38 | $C_6H_5CH_2$ | $C_4H_9CONH$ | $CH_3$ | 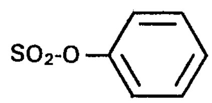 | gelb |
| 39 | $C_2H_5$ | $C_{12}H_{25}O$ | Cl | 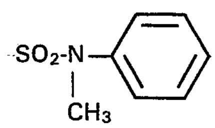 | rot |
| 40 | $OCH_3$ | $CH_3S$ | $CH_3$ | 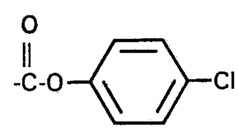 | grün |
| 41 | $C_6H_5CH_2O$ | 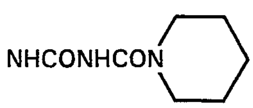 | $CH_3$ | 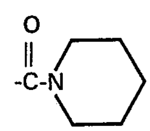 | rot |

*Beispiel 42*

40,5 g Michlers Hydrol und 26,9 g 3-Dimethylamino-phenyl-harnstoff werden in 150 ml Ethanol und 2 ml Eisessig 2 h unter Stickstoff refluxiert. Es wird abgekühlt, abgesaugt und getrocknet: 30,6 g (47% d.Th.) beiges Kristallisat der Formel

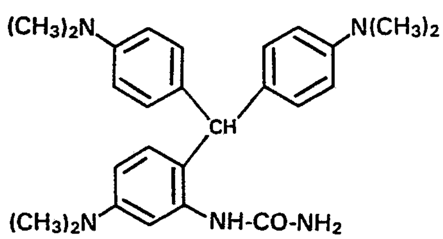

Schmp. aus Tetrachlorkohlenstoff: 118-120°C.

21,5 g dieses Harnstoffs werden in 50 ml Pyridin unter Stickstoff bei 50-60°C suspendiert. Hierzu tropfen während 15 min 8,2 g 2-Ethylhexanoylchlorid. Die entstandene Lösung wird abgekühlt, auf 500 ml Eiswasser gegossen, abgesaugt und mit Wasser gewaschen. Der erhaltene Feststoff wird in 100 ml Ethanol 2 Tage verrührt und abgesaugt. Durch Umkristallisation aus Ethanol erhält man 6,7 g (24% d.Th.) beiges Kristallisat vom Schmp. 203°C und der Formel

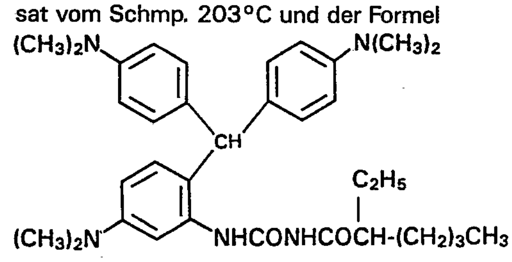

5,6 g dieser Leukoverbindung werden in 10 ml Dimethylformamid bei 60°C mit 2,46 g Chloranil versetzt und 1 h bei dieser Temperatur gehalten. Nach dem Abkühlen wird mit 40 ml Methanol verdünnt, filtriert und auf eine Lösung von 3 g Natriumhydroxid in 250 ml Eiswasser ausgetragen. Die Suspension wird abgesaugt, mit 120 ml Methanol/Wasser 1/2 und Wasser neutral gewaschen. Noch feucht wird in 20 ml Ethanol und 3 ml 10%iger Natronlauge aufgekocht. Nach dem Abkühlen wird abgesaugt, mit Ethanol gewaschen und getrocknet. Man erhält 5,4 g (97% d.Th.) graues Pulver vom Schmp. 226-227°C und der Formel

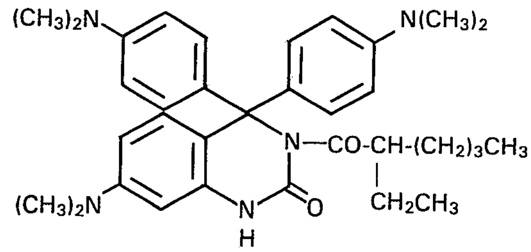

Eine Lösung in Eisessig wird tiefblau mit $\lambda_{max}$ = 606 nm.

Auf Säureton und mit Bisphenol A im Thermodruck wird ebenfalls eine tiefblaue Färbung erzielt.

Analog erhält man die Beispiele 43-51:

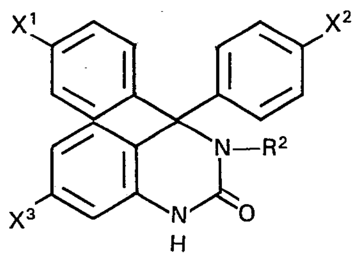

| Bei-spiel | $X^1$ | $X^2$ | $X^3$ | $R^2$ | Farbton auf Säureton oder mit Bisphenol A |
|---|---|---|---|---|---|
| 43 | $N(CH_3)_2$ | $N(CH_3)_2$ | $N(CH_3)_2$ | 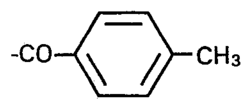 | blau |
| 44 | $N(CH_3)_2$ | $N(CH_3)_2$ | $N(C_2H_5)_2$ | 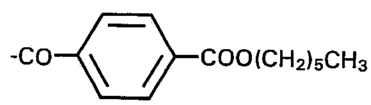 | blau |
| 45 | 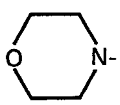 | $OCH_3$ | $N(CH_3)_2$ | 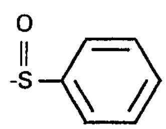 | gelbst. grün |
| 46 | 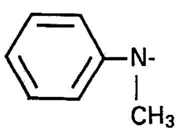 | $OCH_3$ | $N(CH_2\text{-}C_6H_5)_2$ | 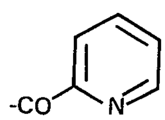 | gelbst. grün |
| 47 | 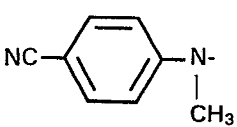 | $OCH_3$ | $N(CH_3)_2$ | 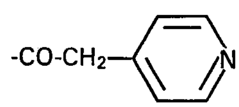 | schwarzblau |
| 48 | $N(CH_3)_2$ | $N(CH_3)_2$ | $N_{CH_2}^{CH_3}CH_2\text{-}CN$ | 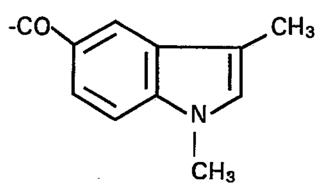 | blau |
| 49 | $N(C_2H_5)_2$ | Cl | $N(CH_2CH_2OCOCH_3)_2$ | $-COCF_3$ | gelbst. grün |

| Beispiel | $X^1$ | $X^2$ | $X^3$ | $R^2$ | Farbton auf Säureton oder mit Bisphenol A |
|---|---|---|---|---|---|
| 50 | $N(CH_3)_2$ | $N(CH_3)_2$ | 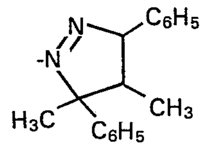 | $-COC_{17}H_{35}$ | grün |
| 51 | 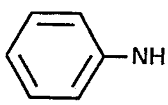 | $SCH_3$ | $N(CH_2CH_2OCH_3)_2$ | 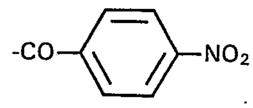 | grün |

*Beispiel 52*

25,7 g 4-Dimethylamino-4'-methoxy-benzhydrol und 44,6 g N-(3-Dimethylaminophenyl)-N'-stearoyl-harnstoff werden in 140 ml Ethanol unter Zusatz von 3 ml Eisessig und 0,7 ml konz. Salzsäure unter Stickstoffatmosphäre 2 h refluxiert. Dann wird abgekühlt und abgesaugt. Man erhält 62,2 g (91 % d.Th.) blassgrünes Kristallisat vom Schmp. 104°C und der Formel

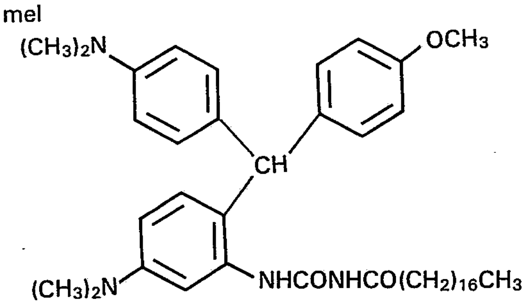

27,4 g dieser Leukoverbindung werden in 60 ml Ethanol bei 60°C mit 9,84 g Chloranil versetzt und 4 h bei dieser Temperatur gerührt. Nach dem Abkühlen wird in 150 ml Methanol aufgenommen, filtriert und auf eine Lösung von 8 g NaOH in 750 ml Eiswasser ausgetragen. Dann wird abgesaugt, mit Wasser neutral gewaschen und noch feucht in 50 ml Ethanol verrührt. Diese Mischung wird auf 100 ml Wasser ausgetragen, abgesaugt und mit Wasser gewaschen. Nach dem Trocknen erhält man 19,5 g (75% d.Th.) beiges Pulver vom Schmp. 91-95°C und der Formel

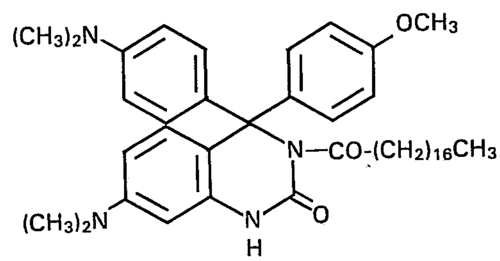

Eine Lösung in Eisessig wird tiefgrün mit $\lambda_{max}$ = 474,621 nm.

Auf Säureton und mit Bisphenol A im Thermodruck wird ebenfalls eine kräftige gelbstichig grüne Färbung erzielt.

Analog lassen sich die folgenden Beispiele herstellen:

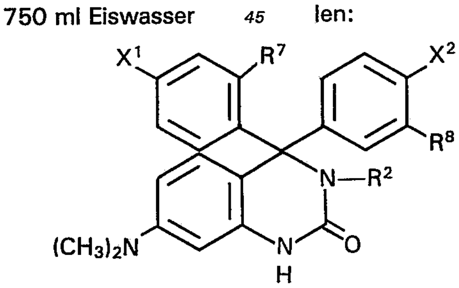

| Beispiel | $X^1$ | $R^7$ | $X^2$ | $R^8$ | $R^2$ | Farbton auf Säureton oder mit Bisphenol A |
|---|---|---|---|---|---|---|
| 53 | $N(CH_3)_2$ | H | 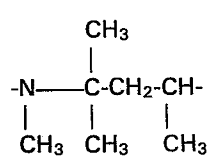 | | 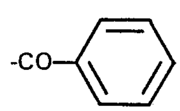 | blau |

| Beispiel | $X^1$ | $R^7$ | $X^2$ | $R^8$ | $R^2$ | Farbton auf Säureton oder mit Bisphenol A |
|---|---|---|---|---|---|---|
| 54 | $N(CH_3)_2$ | $OCH_3$ | $CH_3O$ | H | $-COC(CH_3)_3$ | grün |
| 55 | $CH_3O$ | H | $CH_3O$ | $CH_3O$ | 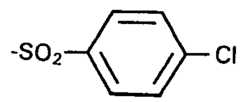 | blaust. rot |
| 56 | $N(C_2H_5)_2$ | H | H | $CH_3$ | $-CO-C_3H_7$ | grün |

*Beispiel 57*

Zu 15,3 g Phosphoroxychlorid und 5,67 g Phosphorpentoxid wird unter Eiskühlung eine Mischung aus 5,36 g Michlers Keton und 8,90 g N-(3-Dimethylaminophenyl)-N'-stearoyl-harnstoff gegeben. Dann wird 26 h bei 20°C und 34 h bei 35°C gerührt. Die zähe Schmelze wird auf 200 g Eis ausgetragen. Mit Natronlauge wird auf pH = 9 gestellt, abgesaugt und neutral gewaschen. Das bläuliche Produkt wird dreimal mit 40 ml Ethanol ausgekocht, jeweils kalt gerührt und abgesaugt. So erhält man 4,45 g (32% d.Th.) der Verbindung des Beispiels 2.

*Beispiel 58*

*Herstellung eines druckempfindlichen Kopierpapiers*

Eine Lösung von 3 g der 4,4-Diaryldihydrochinazolon-Verbindung des Beispiels 2 in 80 g Diisopropylnaphthalin und 17 g Kerosin wird auf an sich bekannte Weise mit Gelatine und Gummiarabicum durch Koazervation mikroverkapselt, mit Stärkelösung vermischt und auf ein Blatt Papier gestrichen. Ein zweites Blatt Papier wird auf der Frontseite mit säureaktiviertem Bentonit als Farbentwickler beschichtet. Das erste Blatt und das mit Farbentwickler beschichtete Blatt werden mit den Beschichtungen benachbart aufeinandergelegt. Durch Schreiben mit der Hand oder mit der Schreibmaschine auf dem ersten Blatt wird Druck ausgeübt, und es entwickelt sich auf dem mit dem Entwickler beschichteten Blatt eine intensive blaue Kopie, die ausgezeichnet lichtecht ist.

*Beispiel 59*

1 g der 4,4-Diaryldihydrochinazolon-Verbindung des Beispiels 16 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1/1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 g/m$^2$ mit einer Mischung — bestehend aus 1 Teil eines Amidwachses, 1 Teil eines Stearinwachses und 1 Teil Zinkchlorid — beschichtet ist. Durch Schreiben mit der Hand oder mit der Schreibmaschine auf dem oberen Blatt wird Druck ausgeübt, und es entwickelt sich auf dem mit dem Farbbildner beschichteten Blatt eine intensive und lichtechte grüne Farbe.

*Beispiel 60*

Man verfährt wie in Beispiel 58, nur setzt man eine Mischung aus 1,5 g der Verbindung des Beispiels 52 und 1,5 g der Verbindung des Beispiels 21 ein. So erhält man ein beschichtetes Blatt, das eine schwarze Kopie liefert, die ausgezeichnet lichtecht ist.

*Herstellung von wärmeempfindlichen Aufzeichnungsmaterialien*

*Beispiel 61*

In einer Kugelmühle werden 32 g 4,4'-Isopropyliden-diphenol (Bisphenol A), 3,8 g Distearylamid des Ethylendiamins, 89 g Koalin, 20 g eines zu 88% hydrolysierten Polyvinylalkohols und 55 ml Wasser gemahlen, bis die Teilchengrösse ca. 5 µm beträgt. In einer 2. Kugelmühle werden 6 g der 4,4-Diaryldihydrochinazolon-Verbindung des Beispiels 1, 3 g eines zu 88% hydrolysierten Polyvinylalkohols und 60 ml Wasser zu einer Teilchengrösse von ca. 3 µm gemahlen. Die beiden Dispersionen werden zusammengegeben und mit einem Trockenauftragsgewicht von 5,5 g/m$^2$ auf ein Papier gestrichen. Durch Berühren des Papiers mit einem erhitzten Kugelschreiber wird eine intensive blaue Farbe erhalten, die eine gute Licht- und Sublimierechtheit hat.

*Beispiel 62*

In einer Kugelmühle werden 2,7 g der 4,4-Diaryldihydrochinazolon-Verbindung des Beispiels 29, 24 g N-Phenyl-N'-(1-hydroxy-2,2,2-trichlor-ethyl)-harnstoff, 16 g Stearinsäureamid, 59 g eines zu 88% hydrolysierten Polyvinylalkohols und 58 ml Wasser gemahlen, bis die Teilchengrösse 2-5 µm beträgt. Diese Suspension wird bei einem Trockenauftragsgewicht von 5,5 g/m$^2$ auf ein Blatt Papier gestrichen. Durch Berührung des Papiers mit einem erhitzten Kugelschreiber wird eine intensive und lichtechte Bordofarbe erhalten.

**Patentansprüche**

1. Chromogene Chinazolone der Formel I

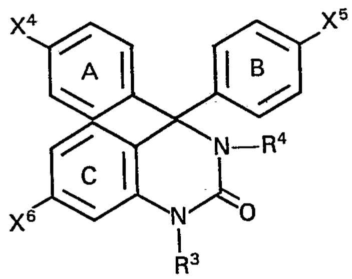

(I),

worin

einer der Reste $X^4$, $X^5$ oder $X^6$ für $NY^4Y^5$ und die anderen unabhängig voneinander Wasserstoff, Halogen, $C_1$- bis $C_{18}$-Alkyl, gegebenenfalls durch Chlor und/oder $C_1$- bis $C_{12}$-Alkyl substituiertes Phenyl, $C_1$- bis $C_{12}$-Alkanoylamino, gegebenanfalls durch Chlor und/oder $C_1$- bis $C_{12}$-Alkyl substituiertes Benzoylamino, gegebenenfalls durch $C_1$- bis $C_4$-Alkyl, $C_1$- bis $C_4$-Alkoxy, Chlor oder Phenyl substituierte Indolyl- oder Piperidylreste, $NY^4Y^5$, $OY^6$ oder $SY^6$,

$R^3$ Wasserstoff, $C_1$- bis $C_{18}$-Alkyl, Cyclohexyl oder gegebenenfalls durch Chlor und/oder $C_1$- bis $C_{18}$-Alkyl substituierte Benzyl- oder Phenylreste,

$R^4$ einen Rest der Formeln

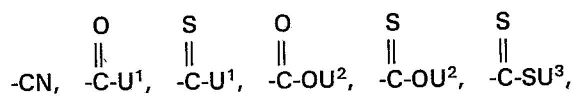

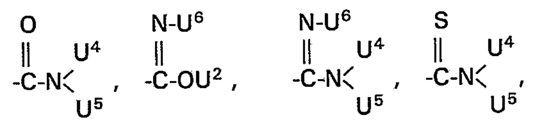

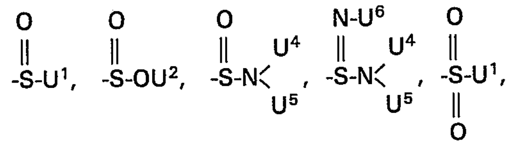

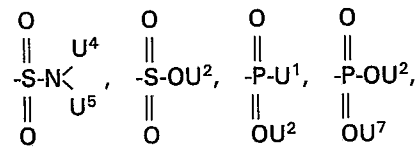

$U^1$ bis $U^7$ Wasserstoff, gegebenenfalls Fluor, Chlor, Cyano, $C_1$- bis $C_4$-Alkoxycarbonyl und/oder $C_1$- bis $C_4$-Alkoxy tragendes $C_1$- bis $C_{30}$-Alkyl, gegebenenfalls Chlor und/oder $C_1$- bis $C_{18}$-Alkyl tragendes Cyclohexyl, gegebenenfalls Chlor, Brom, Nitro, $C_1$- bis $C_{18}$-Alkyl, $C_1$-$C_{18}$-Alkoxy, $C_1$- bis $C_{18}$-Alkylthio, $C_1$-$C_{18}$-Mono- oder -Dialkylamino, $C_1$- bis $C_{18}$-Alkylsulfonyl, Cyano und/oder $C_1$- bis $C_{18}$-Alkoxycarbonyl tragende Phenyl-, Benzyl-, Naphthyl-, Picolyl-, Pyridyl-, Chinolyl-, Pyrimidyl-, Pyrazinyl-, Triazinyl-, Triazolyl-, Thiadiazolyl-, Tetrazolyl-, Indolyl-, gegebenenfalls benzanellierte Imidazol-, Oxazol- oder Thiazolreste,

$Y^4$, $Y^5$ und $Y^6$ unabhängig voneinander Wasserstoff, gegebenenfalls durch Chlor, Cyano, $C_1$- bis $C_4$-Alkoxycarbonyl oder $C_1$- bis $C_4$-Alkoxy substituiertes $C_1$- bis $C_{18}$-Alkyl, Cyclohexyl oder Phenyl oder Benzyl, die durch Chlor, $C_1$- bis $C_{12}$-Alkyl oder $C_1$- bis $C_{12}$- Alkoxy substituiert sein können, durch $C_1$- bis $C_4$-Alkyl substituiertes Piperidyl oder Glieder, die zusammen mit N oder O, an die sie gebunden sind und einem der Ringe A, B oder C zur Vervollständigung eines Ringsystems der folgenden Formeln

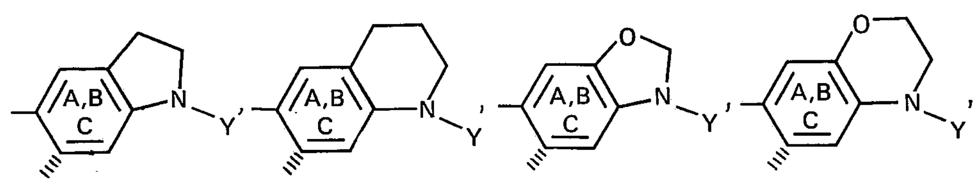

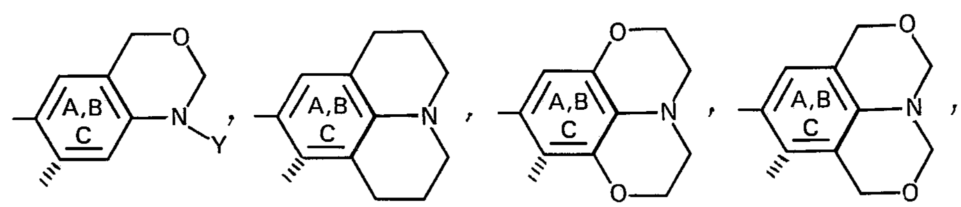

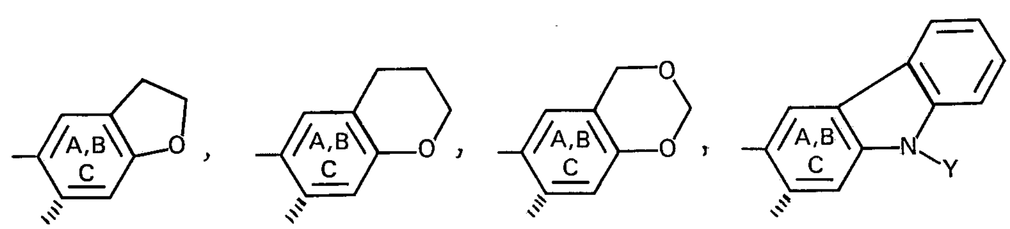

erforderlich sind, bedeuten, worin die gestrichelte Linie die weitere Anellierung im Fall von Ring C bedeutet,

Y für Wasserstoff, $C_1$- bis $C_{18}$-Alkyl, das durch Chlor, Cyano, $C_1$- bis $C_4$-Alkoxycarbonyl oder $C_1$- bis $C_4$-Alkoxy substituiert sein kann, Cyclohexyl, Phenyl oder Benzyl, die durch Chlor, $C_1$- bis $C_{12}$-Alkyl oder $C_1$- bis $C_{12}$-Alkoxy substituiert sein können, steht,

der gesättigte Ringteil bis zu 4 Reste aus der Gruppe Chlor, $C_1$- bis $C_4$-Alkyl, $C_1$- bis $C_4$-Alkoxy oder Phenyl tragen kann,

die Ringe A, B und C durch Chlor, $C_1$- bis $C_4$-Alkyl, $C_1$- bis $C_4$-Alkoxy, gegebenenfalls durch Chlor, $C_1$- bis $C_4$-Alkyl oder $C_1$- bis $C_4$-Alkoxy substituiertes Phenoxy oder Phenylamino und/oder $C_1$- bis $C_4$-Alkanoylamino substituiert sein können, oder

$NY^4Y^5$ einen gegebenenfalls durch Chlor, $C_1$- bis $C_4$-Alkyl oder Phenyl substituierten Pyrrolo-, Pyrrolidino-, Piperidino-, Pipecolino-, Morpholino-, Pyrazolo- oder Pyrazolinorest bedeutet.

2. Chromogene 4,4-Diaryldihydrochinazolone gemäss Anspruch 1 der Formel II

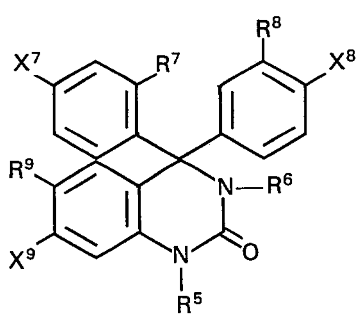

(II),

worin

einer der Reste $X^7$, $X^8$ und $X^9$ für $NY^7Y^8$ steht und die anderen unabhängig voneinander Wasserstoff, Chlor, Brom, $C_1$- bis $C_{18}$-Alkyl, gegebenenfalls durch Chlor und/oder $C_1$- bis $C_4$-Alkyl substituiertes Phenyl, $C_1$- bis $C_4$-Alkanoylamino, gegebenenfalls durch Chlor und/oder $C_1$- bis $C_4$-Alkyl substituiertes Benzoylamino, gegebenenfalls durch Methyl, Ethyl, Methoxy, Chlor oder Phenyl substituierte Indolyl- oder Piperidylreste, $NY^7Y^8$, $OY^9$ oder $SY^9$,

$R^5$ Wasserstoff, $C_1$- bis $C_4$-Alkyl, Cyclohexyl oder Phenyl,

$R^6$ -CN, $-\overset{O}{\overset{\|}{C}}-U^8$, $-\overset{S}{\overset{\|}{C}}-U^8$, $-\overset{O}{\overset{\|}{C}}-OU^9$, $-\overset{O}{\overset{\|}{C}}-N\begin{smallmatrix}U^{10}\\U^{11}\end{smallmatrix}$,

$-\overset{O}{\overset{\|}{S}}-U^8$, $-\underset{O}{\underset{\|}{\overset{O}{\overset{\|}{S}}}}-U^8$, $-\underset{O}{\underset{\|}{\overset{O}{\overset{\|}{S}}}}-OU^9$, $-\underset{O}{\underset{\|}{\overset{O}{\overset{\|}{S}}}}-N\begin{smallmatrix}U^{10}\\U^{11}\end{smallmatrix}$,

$U^8$-$U^{11}$ Wasserstoff, gegebenenfalls Fluor, Chlor oder $C_1$- bis $C_4$-Alkoxy tragendes $C_1$- bis $C_{18}$-Alkyl, Cyclohexyl, gegebenenfalls Chlor und/oder $C_1$- bis $C_8$-Alkyl tragendes Benzyl, gegebenenfalls Chlor, Brom, Nitro, $C_1$- bis $C_{18}$-Alkyl, $C_1$- bis $C_{18}$-Alkoxy, $C_1$- bis $C_{18}$-Alkylthio, $C_1$- bis $C_{18}$-Dialkylamino, $C_1$- bis $C_{18}$-Alkylsulfonyl, Cyano und/oder $C_1$- bis $C_{18}$-Alkoxycarbonyl tragende Phenyl-, Naphthyl-, Picolyl-, Pyridyl-, Chinolyl-, Pyrimidyl-, Pyrazinyl-, Triazinyl-, Triazolyl-, Thiadiazolyl-, Tetrazolyl-, Indolyl-, gegebenenfalls benzanellierte Imidazol-, Oxazol- oder Thiazolreste,

$R^7$, $R^8$ und $R^9$ unabhängig voneinander Wasserstoff, Chlor, Brom, $C_1$- bis $C_4$-Alkyl, $C_1$- bis $C_4$-Alkoxy, $C_1$- bis $C_4$-Alkylthio, Amino oder $C_1$- bis $C_4$-Alkanoylamino, $C_1$- bis $C_4$-Mono- oder -Dialkylamino, gegebenenfalls durch Methyl, Methoxy, Ethoxy oder Chlor substituiertes Anilino oder N-$C_1$- bis $C_4$-Alkylanilino,

$Y^7$, $Y^8$ und $Y^9$ unabhängig voneinander Wasserstoff, durch Chlor, Cyano, Methoxycarbonyl, Hydroxy, Methoxy oder Ethoxy substituiertes $C_1$- bis $C_{18}$-Alkyl; Cyclohexyl, Benzyl oder Phenyl, die durch Chlor, Methyl oder Methoxy substituiert sein können, 2,2,6,6-Tetramethyl- oder 1,2,2,6,6--Pentamethyl-piperidin-4-yl bedeuten oder

$Y^7$, $Y^8$ und $Y^9$ für die Glieder stehen, die zu den in Anspruch 1 unter $Y^4$, $Y^5$ bzw. $Y^6$ aufgeführten Ringsystemen führen oder

$NY^7Y^8$ für einen durch $C_1$- bis $C_4$-Alkyl und/oder Phenyl substituierten Pyrrolidino-, Piperidino-, Morpholino- oder Pyrazolinorest stehen.

3. Chromogene 4,4-Diaryldihydrochinazolone gemäss Anspruch 1 der Formel III

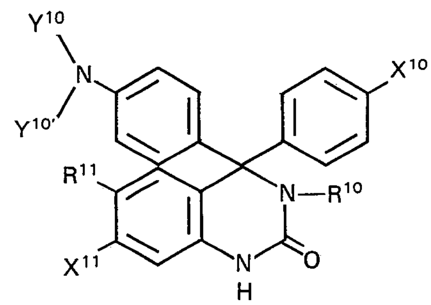

(III),

worin

$R^{10}$ Cyano, Acetyl, Trifluoracetyl, Propionyl, Butanoyl, Pivaloyl, Hexanoyl, Octanoyl, 2-Ethylhexanoyl, Decanoyl, Dodecanoyl, Tetradecanoyl, Hexadecanoyl, Octadecanoyl, Cyclohexylcarbonyl, Phenylacetyl, Benzoyl, Chlorbenzoyl, Toluoyl, tert.-Butyl-benzoyl, Hexylbenzoyl, Dodecylbenzoyl, Anisoyl, Nitrobenzoyl, Cyanobenzoyl, Trifluormethylbenzoyl, Methoxycarbonylbenzoyl, Hexoxycarbonylbenzoyl, Dodecoxycarbonylbenzoyl, Methoxysulfonylbenzoyl, Methylsulfonylbenzoyl, Dinitrobenzoyl, Chlor-nitro-benzoyl, Dichlor-benzoyl, Chlorethylthiobenzoyl, Naphthoyl, Pyridoyl, Chinoloyl, 2-, 3- oder 5-Indoloyl, 5-Benzoxazoloyl, Pyridylacetyl, Methoxycarbonyl, Ethoxycarbonyl, Propoxycarbonyl, Butoxycarbonyl, Hexoxycarbonyl, Dodecoxycarbonyl, Octadecoxycarbonyl, Phenoxycarbonyl, Dimethylaminocarbonyl, Diethylaminocarbonyl, Phenylaminocarbonyl, Methylphenylaminocarbonyl, Dimethylaminosulfonyl, Diethylaminosulfonyl, Phenylaminosulfonyl, Methylphenylaminosulfonyl, Methansulfonyl, Ethansulfonyl, Trifluormethansulfonyl, Nonafluorbutansulfonyl, Heptadecafluoroctansulfonyl, Methansulfinyl, Octansulfinyl, Benzolsulfonyl, Chlorbenzolsulfonyl, Dichlorbenzolsulfonyl, Nitro-benzolsulfonyl, Chlor-nitrobenzolsulfonyl, Trichlor-nitro-benzolsulfonyl, Toluolsulfonyl, Chlor-methyl-benzolsulfonyl, Methylnitro-benzolsulfonyl, Dimethyl-benzolsulfonyl, Methyl-dinitro-benzolsulfonyl, Methoxybenzol-sulfonyl, Dichlor-methoxy-benzolsulfonyl, Methoxynitrobenzolsulfonyl, Naphthalinsulfonyl, Biphenylsulfonyl, Cyano-biphenylsulfonyl, Benzolsulfinyl, Chlorbenzolsulfinyl, Methyl-benzolsulfinyl, Benzoxazol-5-sulfonyl

$R^{11}$ Wasserstoff, Chlor, Methyl oder Methoxy,

$X^{10}$ $N^{11}Y^{11'}$, Wasserstoff, Chlor, $C_1$- bis $C_4$-Alkyl, Methoxy, Ethoxy, Propoxy, Butoxy, Hexoxy, Dodecoxy, Octadecoxy, Benzyloxy, Methylthio oder Ethylthio,

$X^{11}$ Wasserstoff, Chlor, $C_1$- bis $C_4$-Alkyl, Benzyl, Methoxy, Ethoxy, Propoxy, Butoxy, Hexoxy, Dodecoxy, Octadecoxy, Benzyloxy, Methylthio, Ethylthio, Acetylamino, Propionylamino, Butanoylamino, Decanoylamino, Octadecanoylamino, Benzoylamino, $NHCONHR^{10}$ oder $NY^{12}Y^{12'}$ bedeuten,

$Y^{10}$-$Y^{12}$ Methyl, Ethyl, Propyl, Butyl, Hexyl, Dodecyl, Octadecyl, Cyanethyl, Methoxyethyl, Methoxycarbonylethyl, Benzyl, Phenyl, 4-Tolyl, 4-Chlorphenyl, 4-Anisyl, 4-Ethoxy-phenyl, 4-Cyanophenyl,

$Y^{10'}$-$Y^{12'}$ Wasserstoff oder die Bedeutung von $Y^{10}$-$Y^{12}$, oder

$NY^{10}Y^{10'}$, $NY^{11}Y^{11'}$ oder $NY^{12}Y^{12'}$

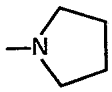 , 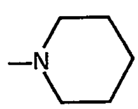 , 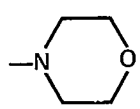 ,

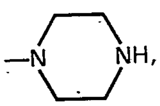 , 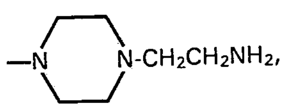 ,

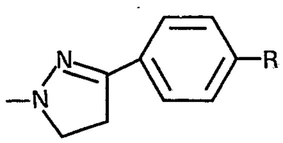 ,

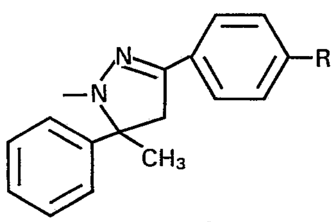 ,

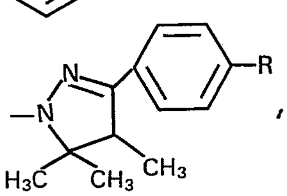 , 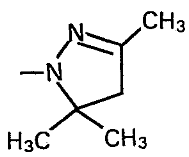

und

R Wasserstoff, Chlor, Methyl, Methoxy, Ethoxy oder Cyano bedeuten.

4. Leukoverbindungen der Formel IV

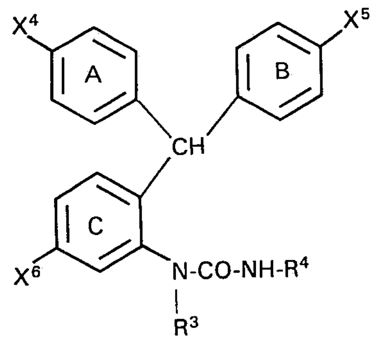

(IV),

worin

$R^3$, $R^4$, $X^4$, $X^5$, $X^6$, A, B und C die in Anspruch 1 angegebene Bedeutung haben.

5. Verfahren zur Herstellung eines 4,4-Diaryl-dihydrochinazolons des Anspruchs 1, dadurch gekennzeichnet, dass man eine Harnstoffverbindung der Formel V

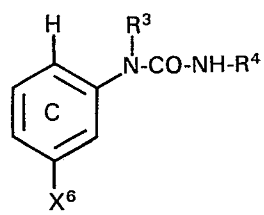

(V)

mit einem Keton der Formel VI

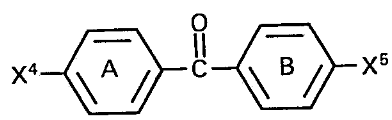

(VI)

umsetzt, worin

$X^4$, $X^5$, $X^6$, $R^3$, $R^4$, A, B und C die oben angegebene Bedeutung besitzen.

6. Verfahren zur Herstellung eines 4,4-Diaryl-dihydrochinazolons des Anspruch 1, dadurch gekennzeichnet, dass man eine Leukoverbindung der Formel IV

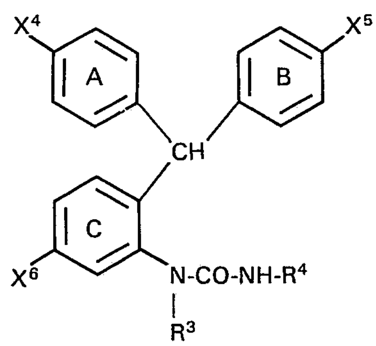

(IV)

oxidiert, worin

$X^4$, $X^5$, $X^6$, $R^3$, $R^4$, A, B und C die in Anspruch 1 angegebene Bedeutung besitzen.

7. Verwendung eines 4,4-Diaryl-dihydrochinazolons der in Anspruch 1 angegebenen Formel als Farbbildner in druck-, wärme- und elektrosensitivem Aufzeichnungsmaterial.

8. Druck-, wärme- und elektrosensitives Aufzeichnungsmaterial, dadurch gekennzeichnet, dass es in seinem Reaktantensystem mindestens eine 4,4-Diaryl-dihydrochinazolonverbindung der in Anspruch 1 angegebenen Formel als Farbbildner enthält.

## Claims

1. Chromogenic quinazolones of the formula I

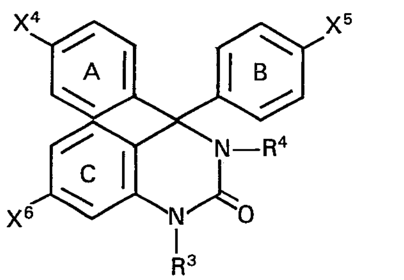

(I)

wherein one of the radicals $X^4$, $X^5$ or $X^6$ stands for $NY^4Y^5$ and the others, independently of each other, denote hydrogen, halogen, $C_1$- to $C_{18}$ -alkyl, optionally chlorine- and/or $C_1$- to $C_{12}$-alkyl-substituted phenyl, $C_1$- to $C_{12}$-alkanoylamino, optionally chlorine and/or $C_1$- to $C_{12}$-alkyl-substituted benzoylamino, optionally $C_1$- to $C_4$-alkyl-, $C_1$- to $C_4$-alkoxy-, chlorine- or phenyl-substituted indolyl or piperidyl radicals, $NY^4Y^5$, $OY^6$ or $SY^6$,

$R^3$ denotes hydrogen, $C_1$- to $C_{18}$-alkyl, cyclohexyl or optionally chlorine- and/or $C_1$- to $C_{18}$-alkyl-substituted benzyl or phenyl radicals,

$R^4$ denotes a radical of the formulae

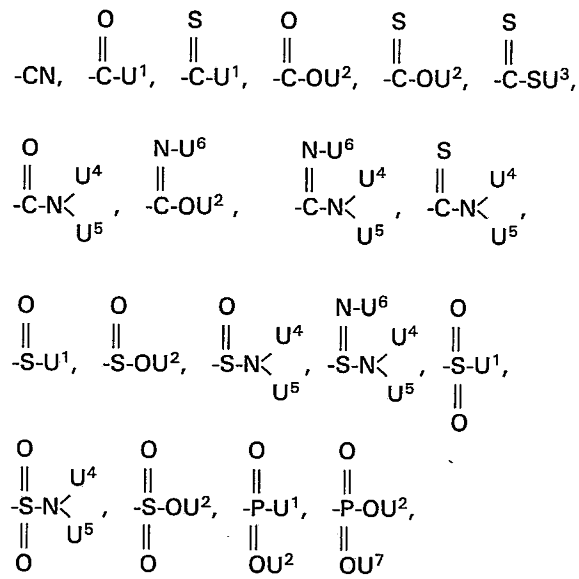

$U^1$ to $U^7$ denote hydrogen, optionally fluorine-, chlorine-, cyano-, $C_1$- to $C_4$-alkoxycarbonyl- and/or $C_1$- to $C_4$-alkoxy-carrying $C_1$- to $C_{30}$-alkyl, optionally chlorine- and/or $C_1$- to $C_{18}$-alkyl-carrying cyclohexyl, optionally chlorine-, bromine-, nitro-, $C_1$- to $C_{18}$-alkyl, $C_1$- to $C_{18}$-alkoxy, $C_1$- to $C_{18}$-alkylthio, $C_1$- to $C_{18}$ -monoalkylamino- or dialkylamino-, $C_1$- to $C_{18}$-alkyl-sulphonyl-, cyano- and/or $C_1$- to $C_{18}$-alkoxycarbonyl-carrying phenyl, benzyl, naphthyl, picolyl, pyridyl, quinolyl, pyrimidyl, pyrazinyl, triazinyl, triazolyl, thiadiazolyl, tetrazolyl, indolyl, optionally benzo-fused imidazole, oxazole or thiazole radicals,

$Y^4$, $Y^5$ and $Y^6$, independently of one another, denote hydrogen, optionally chlorine-, cyano-, $C_1$- to $C_4$-alkoxycarbonyl- or $C_1$- to $C_4$-alkoxy-substituted $C_1$- to $C_{18}$-alkyl, cyclohexyl or phenyl or benzyl, each of which can be substituted by chlorine, $C_1$- to $C_{12}$-alkyl or $C_1$-$C_{12}$ -alkoxy, $C_1$- to $C_4$-alkyl-substituted piperidyl or members which, together with N or O to which they are bonded and one of the rings A, B or C, are necessary for completing a ring system of the following formulae

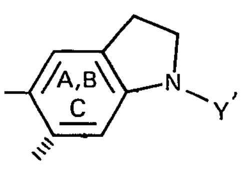 , 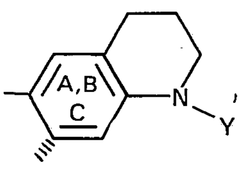 , 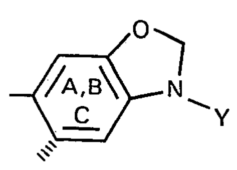 , 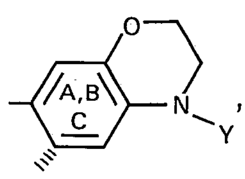 ,

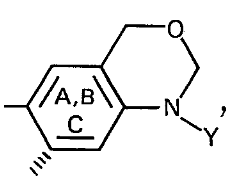 , 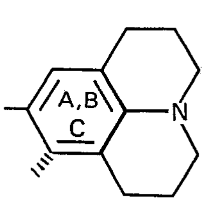 , 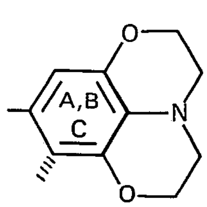 , 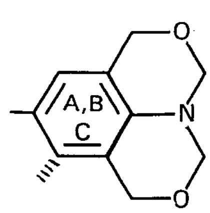 ,

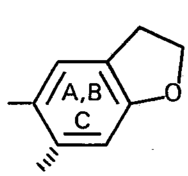 , 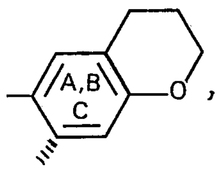 , 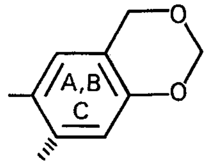 , 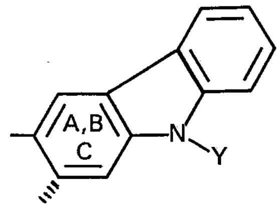

wherein the broken line denotes the further fusion in the case of ring C,

Y stands for hydrogen, $C_1$- to $C_{18}$-alkyl which can be substituted by chlorine, cyano, $C_1$- to $C_4$-alkoxycarbonyl or $C_1$- to $C_4$-alkoxy, cyclohexyl, phenyl or benzyl, each of which can be substituted by chlorine, $C_1$- to $C_{12}$-alkyl or $C_1$- to $C_{12}$-alkoxy,
the saturated ring moiety can carry up to 4 radicals from the group comprising chlorine, $C_1$- to $C_4$-alkyl, $C_1$- to $C_4$-alkoxy and phenyl,
the rings A, B und C can be substituted by chlorine, $C_1$- to $C_4$-alkyl, $C_1$- to $C_4$-alkoxy, optionally chlorine-, $C_1$- to $C_4$-alkyl or $C_1$- to $C_4$-alkoxy-substituted phenoxy or phenylamino and/or $C_1$- to $C_4$-alkanoylamino,
or $NY^4Y^5$ denotes an optionally chlorine-, $C_1$- to $C_4$-alkyl- or phenyl-substituted pyrrolo, pyrrolidino, piperidino, pipecolino, morpholino, pyrazolo or pyrazolino radical.

2. Chromogenic 4,4-diaryldihydroquinazolones according to Claim 1 of the formula II

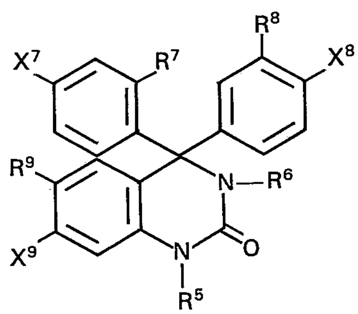

(II)

wherein one of the radicals $X^7$, $X^8$ and $X^9$ stands for $NY^7Y^8$ and the others, independently of each other, denote hydrogen, chlorine, bromine, $C_1$- to $C_{18}$-alkyl, optionally chlorine- and/or $C_1$- to $C_4$-alkyl-substituted phenyl, $C_1$- to $C_4$-alkanoylamino, optionally chlorine- and/or $C_1$- to $C_4$-alkyl-substituted benzoylamino, optionally methyl-, ethyl-, methoxy-, chlorine- or phenyl-substituted indolyl or piperidyl radicals, $NY^7Y^8$, $OY^9$ or $SY^9$,

$R^5$ denotes hydrogen, $C_1$- to $C_4$-alkyl, cyclohexyl or phenyl,

$R^6$ denotes

$R^6$ -CN, 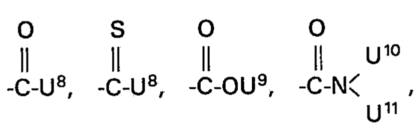

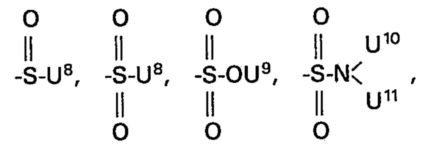

$U^8$-$U^{11}$ denote hydrogen, optionally fluorine-, chlorine- or $C_1$- to $C_4$-alkoxy-carrying $C_1$- to $C_{18}$-alkyl, cyclohexyl, optionally chlorine- and/or $C_1$- to $C_8$-alkyl-carrying benzyl, optionally chlorine-, bromine-, nitro-, $C_1$- to $C_{18}$-alkyl, $C_1$- to $C_{18}$-alkoxy, $C_1$- to $C_{18}$-alkylthio, $C_1$- to $C_{18}$-dialkylamino, $C_1$- to $C_{18}$-alkylsulphonyl, cyano- and/or $C_1$- to $C_{18}$-alkoxycarbonyl-carrying phenyl, naphthyl, picolyl, pyridyl, quinolyl, pyrimidyl, pyrazinyl, triazinyl, triazolyl, thiadiazolyl, tetrazolyl, indolyl, optionally benzo-fused imidazole, oxazole or thiazole radicals,

$R^7$, $R^8$ and $R^9$, independently of one another, denote hydrogen, chlorine, bromine, $C_1$- to $C_4$-alkyl, $C_1$- to $C_4$-alkoxy, $C_1$- to $C_4$-alkylthio, amino or $C_1$- to $C_4$-alkanoylamino, $C_1$- to $C_4$-monoalkylamino or -dialkylamino, optionally methyl-, methoxy-, ethoxy- or chlorine-substituted anilino or N- $C_1$- to $C_4$-alkylamino,

$Y^7$, $Y^8$ and $Y^9$, independently of one another, denote hydrogen, chlorine-, cyano-, methoxycarbonyl-, hydroxyl-, methoxy- or ethoxy-substituted $C_1$- to $C_{18}$-alkyl; cyclohexyl, benzyl or phenyl, each of which can be substituted by chlorine, methyl or methoxy, 2,2,6,6-tetramethylpiperidin-4-yl or 1,2,2,6,6-pentamethylpiperidin-4-yl or

$Y^7$, $Y^8$ and $Y^9$ stand for the members which lead to the ring system listed in claim 1 under $Y^4$, $Y^5$ and $Y^6$ or

$NY^6Y^8$ stands for a $C_1$- to $C_a$-alkyl- and/or phenyl-substituted pyrrolidino, piperidino, morpholino or pyrazolino radical.

3. Chromogenic 4,4-diaryldihydroquinazolones according to Claim 1 of the formula III

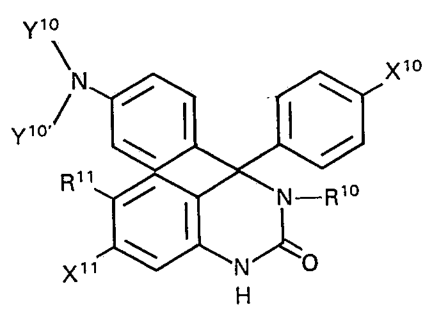

(III)

wherein $R^{10}$ denotes cyano, acetyl, trifluoracetyl, propionyl, butanoyl, pivaloyl, hexanoyl, octanoyl,

2-ethylhexanoyl, decanoyl, dodecanoyl, tetradecanoyl, hexadecanoyl, octadecanoyl, cyclohexylcarbonyl, phenylacetyl, benzoyl, chlorobenzoyl, toluol, tert.-butylbenzoyl, hexylbenzoyl, dodecylbenzoyl, anisoyl, nitrobenzoyl, cyanobenzoyl, trifluoromethylbenzoyl, methoxycarbonylbenzoyl, hexoxycarbonylbenzoyl, dodecoxycarbonylbenzoyl, methoxysulphonylbenzoyl, methylsulphonylbenzoyl, dinitrobenzoyl, chloronitrobenzoyl, dichlorobenzoyl, chloroethylthiobenzoyl, naphthoyl, pyridoyl, quinoloyl, 2-, 3- or 5-indolyl, 5-benzoxazolyl, pyridyl-acetyl, methoxycarbonyl, ethoxycarbonyl, propoxycarbonyl, butoxycarbonyl, hexoxycarbonyl, dodecoxycarbonyl, octadecoxycarbonyl, phenoxycarbonyl, dimethylaminocarbonyl, diethylaminocarbonyl, phenylaminocarbonyl, methylphenylaminocarbonyl, dimethylaminosulphonyl, diethylaminosulphonyl, phenylaminosulphonyl, methylphenylaminosulphonyl, methanesulphonyl, ethanesulphonyl, trifluoromethanesulphonyl, nonafluorobutanesulphonyl, heptadecafluorooctanesulphonyl, methanesulphinyl, octanesulphinyl, benzenesulphonyl, chlorobenzenesulphonyl, dichlorobenzenesulphonyl, nitrobenzenesulphonyl, chloronitrobenzenesulphonyl, trichloronitrobenzenesulphonyl, toluenesulphonyl, chloromethylbenzenesulphonyl, methylnitrobenzenesulphonyl, dimethylbenzenesulphonyl, methyldinitrobenzenesulphonyl, methoxybenzenesulphonyl, dichloromethoxybenzenesulphonyl, methoxynitrobenzenesulphonyl, naphthalenesulphonyl, biphenylsulphonyl, cyanobiphenylsulphonyl, benzenesulphinyl, chlorobenzenesulphinyl, methylbenzenesulphinyl, benzoxazole-5-sulphonyl,

$R^{11}$ denotes hydrogen, chlorine, methyl or methoxy,

$X^{10}$ denotes $NY^{11}Y^{11'}$, hydrogen, chlorine, $C_1$- to $C_4$-alkyl, methoxy, ethoxy, propoxy, butoxy, hexoxy, dodecoxy, octadecoxy, benzyloxy, methylthio or ethylthio,

$X^{11}$ denotes hydrogen, chlorine, $C_1$- to $C_4$-alkyl, benzyl, methoxy, ethoxy, propoxy, butoxy, hexoxy, dodecoxy, octadecoxy, benzyloxy, methylthio, ethylthio, acetylamino, propionylamino, butanoylamino, decanoylamino, octadecanoylamino, benzoylamino, $NHCONHR^{10}$ or $NY^{12}Y^{12}$,

$Y^{10}$-$Y^{12}$ denote methyl, ethyl, propyl, butyl, hexyl, dodecyl, octadecyl, cyanoethyl, methoxyethyl, methoxycarbonylethyl, benzyl, phenyl, 4-tolyl, 4-chlorophenyl, 4-anisyl, 4-ethoxyphenyl, 4-cyanophenyl,

$Y^{10'}$-$Y^{12'}$ denote hydrogen or the meaning of $Y^{10}$-$Y^{12}$ or

$NY^{10}Y^{10'}$, $NY^{11}$-$Y^{11'}$, or $NY^{12}Y^{12'}$ denotes

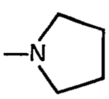 , 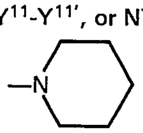 , 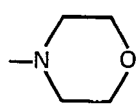 ,

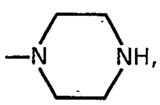

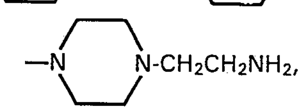

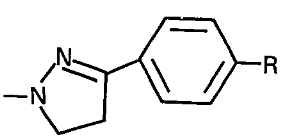 ,

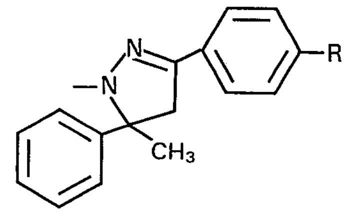 ,

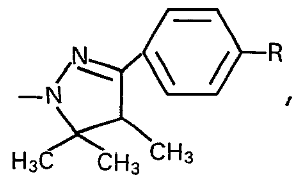 , 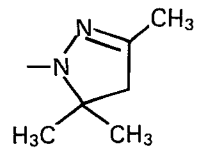

and

R denotes hydrogen, chlorine, methyl, methoxy, ethoxy or cyano.

4. Leuco compounds of the formula IV

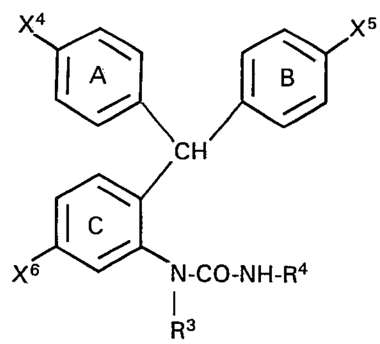

(IV)

wherein

$R^3$, $R^4$, $X^4$, $X^5$, $X^6$, A, B and C have the meaning mentioned in Claim 1.

5. Process for preparing a 4,4-diaryldihydroquinazolone of Claim 1, characterized in that a urea compound of the formula V

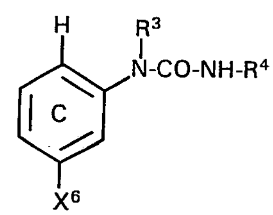

(V)

is reacted with a ketone of the formula VI

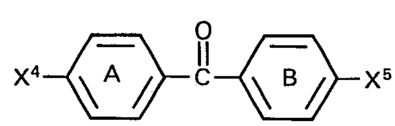

(VI)

wherein

$X^4$, $X^5$, $X^6$, $R^4$, $R^5$, A, B and C have the meaning mentioned above.

6. Process for preparing a 4,4-diaryldihydroquinazolone of Claim 1, characterized in that a leuco compound of the formula IV

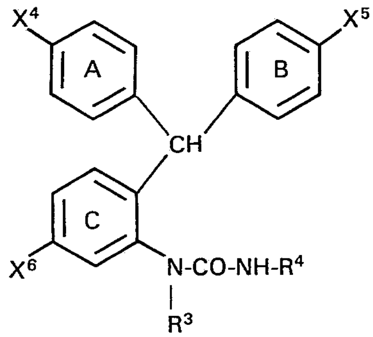

(IV)

wherein

$X^4$, $X^5$, $X^6$, $R^3$, $R^4$, A, B and C have the meaning mentioned in Claim 1, is oxidized.

7. Use of a 4,4-diaryldihydroquinazolone of the formula indicated in Claim 1, as a colour former in pressure-, heat- and electro-sensitive recording material.

8. Pressure-, heat- and electro-sensitive recording material, characterized in that it contains in its reaction system at least one 4,4-diaryldihydroquinazolone compound of the formula indicated in Claim 1 as a colour former.

**Revendications**

1. Quinazolones chromogènes de formule I

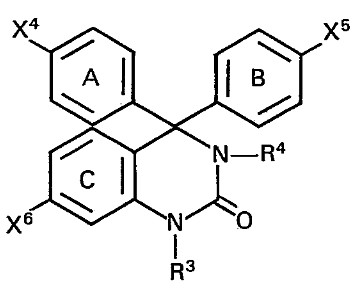

(I)

dans laquelle

l'un des restes $X^4$, $X^5$ ou $X^6$ représente un groupe $NY^4Y^5$ et les autres représentent indépendamment l'un de l'autre l'hydrogène, un halogène, un groupe alkyle en $C_1$ à $C_{18}$, un groupe phényle éventuellement substitué par du chlore et/ou par un radical alkyle en $C_1$ à $C_{12}$, un groupe alcanoylamino en $C_1$ à $C_{12}$, un groupe benzoylamino éventuellement substitué par du chlore et/ou par un radical alkyle en $C_1$ à $C_{12}$, des restes indolyle ou pipéridyle éventuellement substitués par un radical alkyle en $C_1$ à $C_4$, alkoxy en $C_1$ à $C_4$, du chlore ou un radical phényle, un groupe $NY^4Y^5$, $OY^6$ ou $SY^6$,

$R^3$ représente l'hydrogène, un groupe alkyle en $C_1$ à $C_{18}$, un groupe cyclohexyle ou des restes benzyle ou phényle éventuellement substitués par du chlore et /ou par un radical alkyle en $C_1$ à $C_{18}$,

$R^4$ est un reste de formules

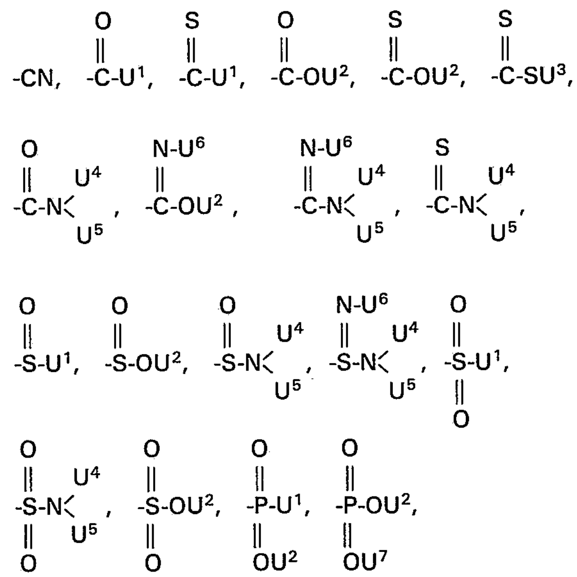

$U^1$ à $U^7$ représentent de l'hydrogène, un groupe alkyle en $C_1$ à $C_{30}$ portant éventuellement du fluor, du chlore, un radical cyano (alkoxy en $C_1$ à $C_4$) carbonyle et/ou alkoxy en $C_1$ à $C_4$, un groupe cyclohexyle portant éventuellement du chlore et/ou un radical alkyle en $C_1$ à $C_{18}$, des restes phényle, benzyle, naphthyle, picolyle, pyridyle, quinolyle, pyrimidyle, pyrazinyle, triazinyle, triazolyle, thiadiazolyle, tétrazolyle, indolyle, imidazole éventuellement condensé au benzène, octazole ou thiazole portant le cas échéant du chlore, du brome, un radical nitro, alkyle en $C_1$ à $C_{18}$, alkoxy en $C_1$ à $C_{18}$, alkylthio rn $C_1$ à $C_{18}$, mono- ou di(alkyle en $C_1$ à $C_{18}$)amino, alkylsulfonyle en $C_1$ à $C_{18}$, cyano et/ou (alkoxy en $C_1$ à $C_{18}$)-carbonyle,

$Y^4$, $Y^5$ et $Y^6$ représentent indépendamment l'un de l'autre de l'hydrogène, un reste alkyle en $C_1$ à $C_{18}$ éventuellement substitué par du chlore, un radical cyano, (alkoxy en $C_1$ à $C_4$) carbonyle, ou alkoxy en $C_1$ à $C_4$, un reste cyclohexyle ou des restes phényle ou benzyle qui peuvent être substitués par du chlore, un radical alkyle en $C_1$ à $C_{12}$ ou alkoxy en $C_1$ à $C_{12}$, un reste pipéridyle substitué par un radical alkyle en $C_1$ à $C_4$ ou désignent des membres qui sont nécessaires, conjointement avec les atomes N ou O, auxquels ils sont liés et avec l'un des noyaux A, B ou C, pour compléter un système cyclique des formules suivantes:

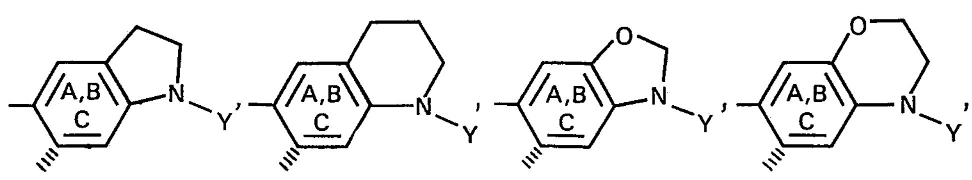

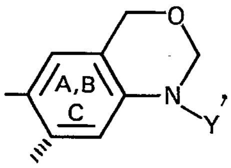,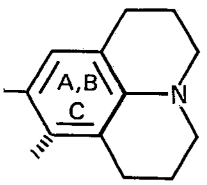,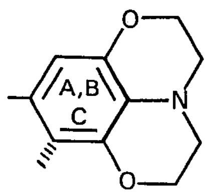,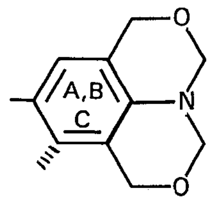,

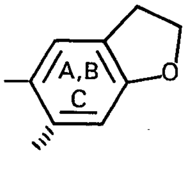,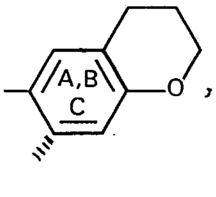,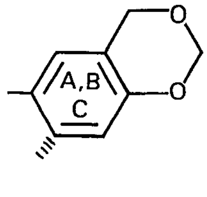,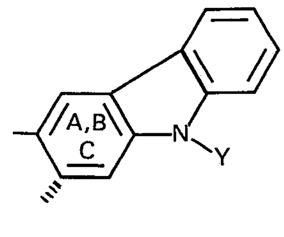

où la ligne en traits interrompus désigne la nouvelle condensation dans le cas du noyau C,

Y représente l'hydrogène, un groupe alkyle en $C_1$ à $C_{18}$ qui peut être substitué par du chlore, un radical cyano, (alkoxy en $C_1$ à $C_4$)-carbonyle ou alkoxy en $C_1$ à $C_4$, des restes cyclohexyle, phényle ou benzyle qui peuvent être substitués par du chlore, un radical alkyle en $C_1$ à $C_{12}$ ou un radical alkoxy en $C_1$ à $C_{12}$, la partie saturée du noyau peut porter jusqu'à 4 restes du groupe chlore, alkyle en $C_1$ à $C_4$, alkoxy en $C_1$ à $C_4$ ou phényle,

les noyaux A, B et C peuvent être substitués par du chlore, un groupe alkyle en $C_1$ à $C_4$, alkoxy en $C_1$ à $C_4$, phénoxy ou phénylamino éventuellement substitué par du chlore ou un radical alkyle en $C_1$ à $C_4$ ou alkoxy en $C_1$ à $C_4$ et/ou un groupe alcanoylamino en $C_1$ à $C_4$, ou bien

$NY^4Y^5$ représente un reste pyrrolo, pyrrolidino, pipéridino, pipécolino, morpholino, pyrazolo ou pyrazolino, éventuellement substitué par du chlore, un radical alkyle en $C_1$ à $C_4$ ou phényle.

2. 4,4-diaryldihydroquinazolones chromogènes suivant la revendication 1, de formule II

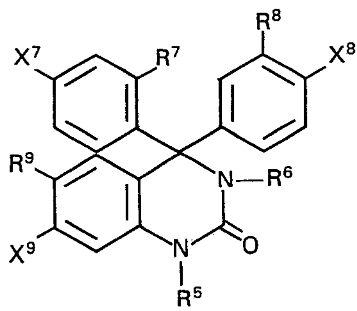

(II)

dans laquelle

l'un des restes $X^7$, $X^8$ et $X^9$ représente un groupe $NY^7Y^8$ et les autres représentent, indépendamment, l'hydrogène, le chlore, le brome, un groupe alkyle en $C_1$ à $C_{18}$, un groupe phényle éventuellement substitué par du chlore et/ou par un radical alkyle en $C_1$ à $C_4$, un groupe alcanoylamino en $C_1$ à $C_4$, un groupe benzoylamino éventuellement substitué par du chlore et/ou par un radical alkyle en $C_1$ à $C_4$, des restes indolyle ou pipéridyle éventuellement substitués par un radical méthyle, éthyle, méthoxy, chloro ou phényle, un groupe $NY^7Y^8$, $OY^9$ ou $SY^9$,

$R^5$ représente l'hydrogène, un groupe alkyle en $C_1$ à $C_4$, cyclohexyle ou phényle,

$R^6$ représente

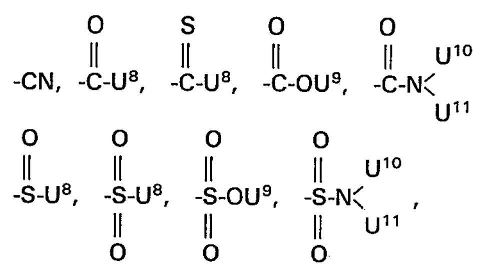

$U^8$-$U^{11}$ représentent l'hydrogène, un groupe alkyle en $C_1$ à $C_{18}$ portant éventuellement du fluor, du chlore ou un radical alkoxy en $C_1$ à $C_4$, un groupe cyclohexyle, un groupe benzyle portant éventuellement du chlore et/ou un radical alkyle en $C_1$ à $C_8$, des restes phényle, naphthyle, picolyle, pyridyle, quinolyle, pyrimidyle, pyrazinyle, triazinyle, triazolyle, thiadiazolyle, tétrazolyle, indolyle, imidazole éventuellement condensé à du benzène, oxazole ou thiazole portant le cas échéant du chlore, du brome, un radical nitro, alkyle en $C_1$ à $C_{18}$, alkoxy en $C_1$ à $C_{18}$, alkylthio en $C_1$ à $C_{18}$, di(alkyle en $C_1$ à $C_{18}$)amino, alkylsulfonyle en $C_1$ à $C_{18}$, cyano et/ou (alkoxy en $C_1$ à $C_{18}$) carbonyle,

$R^7$, $R^8$ et $R^9$ représentent indépendamment l'un de l'autre de l'hydrogène, du chlore, du brome, un grouoe alkyle en $C_1$ à $C_4$, alkoxy en $C_1$ à $C_4$, alkylthio en $C_1$ à $C_4$, amino ou alcanoylamino en $C_1$ à $C_4$, mono- ou di(alkyle en $C_1$ à $C_4$)-amino, anilino éventuellement substitué par un radical méthyle, méthoxy, éthoxy ou chloro, ou un groupe N-(alkyle en $C_1$ à $C_4$)anilino,

$Y^7$, $Y^8$ et $Y^9$ représentent indépendamment l'un de l'autre de l'hydrogène, un groupe alkyle en $C_1$ à $C_{18}$ éventuellement substitué par un radical chloro, cyano, méthoxycarbonyle, hydroxy, méthoxy ou éthoxy; des restes cyclohexyle, benzyle ou phényle qui peuvent être substitués par du chlore, un radical méthyle ou méthoxy, des restes 2,2,6,6--tétraméthyl- ou 1,2,2,6,6-pentaméthylpipéridine--4-yle ou

$Y^7$, $Y^8$ et $Y^9$ représentent des membres qui mènent aux systèmes cycliques indiqués pour $Y^4$, $Y^5$ et respectivement $Y^6$ dans la revendication 1, ou bien

$NY^7Y^8$ représentent un reste pyrrolidino, pipéridino, morpholino ou pyrazolino substitué par un radical alkyle en $C_1$ à $C_4$ et/ou phényle.

3. 4,4-diaryldihydroquinazolones chromogènes selon la revendication 1, de formule III

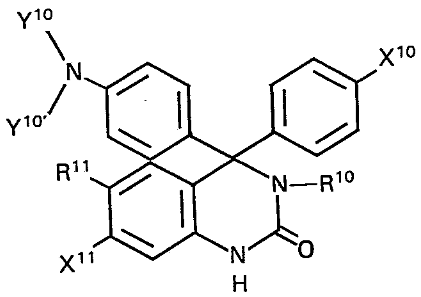

(III)

dans laquelle

$R^{10}$ est un groupe cyano, acétyle, trifluoracétyle, propionyle, butanoyle, pivaloyle, hexanoyle, octanoyle, 2-éthylhexanoyle, décanoyle, dodécanoyle, tétradécanoyle, hexadécanoyle, octadécanoyle, cyclohexylcarbonyle, phénylacétyle, benzoyle, chlorobenzoyle, toluoyle, tertio-butylbenzoyle, hexylbenzoyle, dodécylbenzoyle, anisoyle, nitrobenzoyle, cyanobenzoyle, trifluorométhylbenzoyle, méthoxycarbonylbenzoyle, hexoxycarbonylbenzoyle, dodécoxycarbonylbenzoyle, méthoxysulfonylbenzoyle, méthylsulfonylbenzoyle, dinitrobenzoyle, chloronitrobenzoyle, dichlorobenzoyle, chloréthylthiobenzoyle, naphtoyle, pyridoyle, quinoloyle, 2-, 3- ou 5-indoloyle, 5-benzoxazoloyle, pyridylacétyle, méthoxycarbonyle, éthoxycarbonyle, propoxycarbonyle, butoxycarbonyle, hexoxycarbonyle, dodécoxycarbonyle, octadécoxycarbonyle, phénoxycarbonyle, diméthylaminocarbonyle, diéthylaminocarbonyle, phénylaminocarbonyle, méthylphénylaminocarbonyle, diméthylaminosulfonyle, diéthylaminosulfonyle, phénylaminosulfonyle, méthylphénylaminosulfonyle, méthanesulfonyle, éthanesulfonyle, trifluorométhanesulfonyle, nonafluorobutanesulfonyle, heptadécafluoroctanesulfonyle, méthanesulfinyle, octanesulfinyle, benzènesulfonyle, chlorobenzènesulfonyle, dichlorobenzènesulfonyle, nitrobenzènesulfonyle, chloronitrobenzènesulfonyle, trichloronitrobenzènesulfonyle, toluènesulfonyle, chlorométhylbenzènesulfonyle, méthylnitrobenzènesulfonyle, diméthylbenzènesulfonyle, méthyldinitrobenzènesulfonyle, méthoxybenzènesulfonyle, dichlorométhoxybenzènesulfonyle, méthoxynitrobenzènesulfonyle, naphtalènesulfonyle, biphénylsulfonyle, cyanobiphenylsulfonyle, benzènesulfinyle, chlorobenzènesulfinyle, méthylbenzènesulfinyle, benzoxazole-5-sulfonyle

$R^{11}$ est l'hydrogène, le chlore, un groupe méthyle ou méthoxy,

$X^{10}$ est un groupe $NY^{11}Y^{11'}$, l'hydrogène, le chlore, un groupe alkyle en $C_1$ à $C_4$, méthoxy, éthoxy, propoxy, butoxy, hexoxy, dodécoxy, octadécoxy, benzyloxy, méthylthio ou éthylthio,

$X^{11}$ est l'hydrogène, le chlore, un groupe alkyle en $C_1$ à $C_4$, benzyle, méthoxy, éthoxy, propoxy, butoxy, hexoxy, dodécoxy, octadécoxy, benzyloxy, méthylthio, éthylthio, acétylamino, propionylamino, butanoylamino, décanoylamino, octadécanoylamino, benzoylamino, $NHCONHR^{10}$ ou $NY^{12}Y^{12'}$,

$Y^{10}$-$Y^{12}$ sont des groupes méthyle, éthyle, propyle, butyle, hexyle, dodécyle, octadécyle, cyanéthyle, méthoxyéthyle, méthoxycarbonyléthyle, benzyle, phényle, 4-tolyle, 4-chlorophényle, 4-anisyle, 4-éthoxyphényle, 4-cyanophényle,

$Y^{10'}$-$Y^{12'}$ représentent l'hydrogène ou ont la définition de $Y^{10}$-$Y^{12}$, ou bien

$NY^{10}Y^{10'}$, $NY^{11}Y^{11'}$ ou $NY^{12}Y^{12'}$ représentent

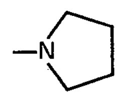 , 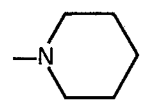 , 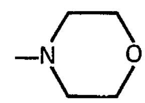 ,

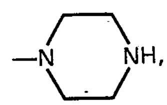 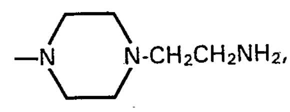

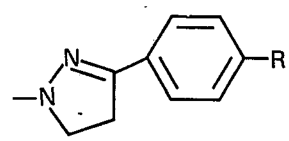 ,

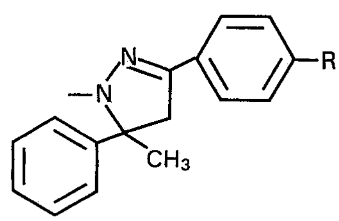 ,

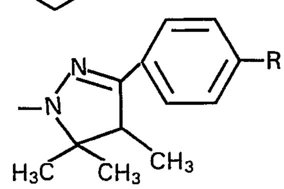 , 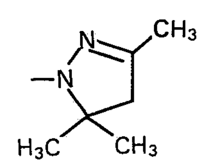

et

R représente l'hydrogène, le chlore, un groupe méthyle, méthoxy, éthoxy ou cyano.

4. Leucodérivés de formule IV

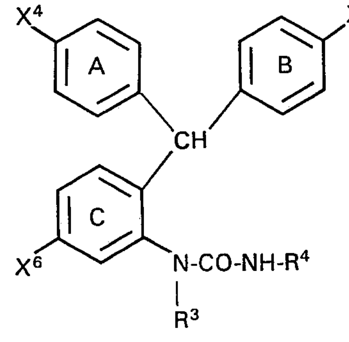

(IV)

dans laquelle

$R^3$, $R^4$, $X^4$, $X^5$, $X^6$, A, B et C ont la définition indiquée dans la revendication 1.

5. Procédé de préparation d'une 4,4-diaryldihydroquinazolone suivant la revendication 1, caractérisé en ce qu'on fait réagir un dérivé d'urée de formule V

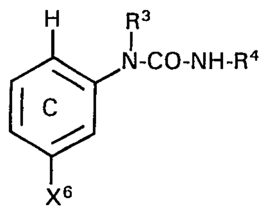

(V)

avec une cétone de formule VI

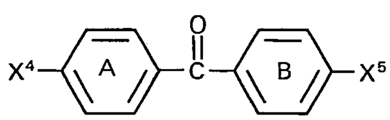

(VI)

formules dans lesquelles

$X^4$, $X^3$, $X^6$, $R^4$, $R^5$, A, B et C ont la définition indiquée ci-dessus.

6. Procédé de préparation d'une 4,4-diaryldihydroquinazolone suivant la revendication 1, caractérisé en ce qu'on oxyde un leucodérivé de formule

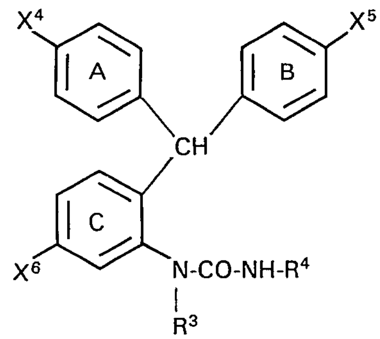

(IV)

dans laquelle

$X^4$, $X^5$, $X^6$, $R^3$, $R^4$, A, B et C ont la définition indiquée dans la revendication 1.

7. Utilisation d'une 4,4-diaryldihydroquinazolone de formule indiquée dans la revendication 1 comme chromogène dans un support d'enregistrement sensible à la pression, à la chaleur et à l'électricité.

8. Support d'enregistrement sensible à la pression, à la chaleur et à l'électricité, caractérisé en ce qu'il contient comme chromogène dans son système de corps réactionnels au moins une 4,4-diaryldihydroquinazolone de formule indiquée dans la revendication 1.